(19) Europäisches Patentamt
European Patent Office
Office européen des brevets

(11) **EP 1 862 156 B1**

(12) **EUROPEAN PATENT SPECIFICATION**

(45) Date of publication and mention
of the grant of the patent:
**02.01.2013 Bulletin 2013/01**

(21) Application number: **06729464.5**

(22) Date of filing: **20.03.2006**

(51) Int Cl.:
*A61F 13/49* (2006.01)     *A61F 13/53* (2006.01)
*A61F 13/15* (2006.01)     *A61F 13/494* (2006.01)
*A61F 13/539* (2006.01)     *A61F 13/515* (2006.01)

(86) International application number:
**PCT/JP2006/305496**

(87) International publication number:
**WO 2006/101061 (28.09.2006 Gazette 2006/39)**

(54) **ABSORBENT ARTICLES**

SAUGFÄHIGE ARTIKEL

ARTICLES ABSORBANTS

(84) Designated Contracting States:
**DE FR GB SE**

(30) Priority: 23.03.2005  JP 2005082909
23.03.2005  JP 2005082961
24.03.2005  JP 2005085108
24.03.2005  JP 2005087111

(43) Date of publication of application:
**05.12.2007   Bulletin 2007/49**

(60) Divisional application:
**12182517.8 / 2 537 498**

(73) Proprietor: **Kao Corporation**
**Chuo-ku,**
**Tokyo 103-8210 (JP)**

(72) Inventors:
• **KOUTA, Takuya c/o Kao Corporation, Research Lab.,**
**Tochigi 3213497 (JP)**

• **KASAI, Takao c/o Kao Corporation, Research Lab.,**
**Tochigi 3213497 (JP)**
• **KATOH, Takahiro c/o Kao Corporation, Research Lab.**
**Tochigi 3213497 (JP)**
• **NIINOMI, Masahiko c/o Kao Corp., Research Lab.**
**Tochigi 3213497 (JP)**

(74) Representative: **Vossius & Partner**
**Siebertstrasse 4**
**81675 München (DE)**

(56) References cited:
**EP-A1- 1 417 946      EP-A2- 1 323 400**
**JP-A- 04 504 285      JP-A- 09 273 037**
**US-A- 5 486 167       US-A1- 2003 134 559**

## Description

Technical Field:

The present invention relates to an absorbent article such as a disposable diaper, a sanitary napkin, and an incontinence pad.

Background Art:

Absorbent members for absorbent articles using an opened tow of continuous filaments are known. Included is an absorbent member comprising a crimped cellulose acetate fiber tow layer and a ground pulp layer superposed on one side of the tow layer, the two layers being united by pressing in the thickness direction (see JP-A-57-160457). The absorbent member is described as having improved body fluid distribution. However, because cellulose acetate fiber is inferior in water absorption to pulp, a large quantity of ground pulp should be used in combination to secure increased absorption capacity. As a result, the absorbent member has an increased thickness, which reduces wearing comfort of the absorbent article.

WO 2001/34082 proposes an absorbent core composed of an upper layer, a lower layer, and an absorbent layer interposed therebetween. The absorbent layer includes a superabsorbent polymer sprinkled layer on which a fiber layer made of a cellulose acetate fiber tow is disposed. The superabsorbent polymer has a part thereof bonded to the lower layer with an adhesive and another part thereof entering the fiber tow layer. Although part of the superabsorbent polymer enters the fiber tow layer, the most part of the superabsorbent polymer is bonded to the lower layer. In other words, the fiber tow layer and the superabsorbent polymer sprinkled layer are independent of each other so that the structure of the absorbent core tends to be destroyed when deformed during use by the wearer's movement.

Among known absorbent layers having a tow of continuous filaments is the one disclosed in JP-A-2001-276125, in which the tow extends in the thickness direction of the absorbent layer. The publication says that excrement moves downward through the interstices in the tow and draws away from the wearer's skin and is thus prevented from causing overhydration or rash. In order for the absorbent layer to have such a structure, the tow should have some length so that the absorbent layer is of necessity thick.

Apart from these techniques, Applicant previously proposed in EP1417946A1 an absorbent article having an absorbent member containing crimped fiber. According to the disclosure, the absorbent member has less bunching, increased absorptivity, and improved comfort and fit while worn. Staple fiber is usually used as the crimped fiber.

Separately from the above-described absorbent articles, pull-on disposable diapers having extensible side panels are known (see, e.g., EP 0320989A2). Pull-on disposable diapers of this type are apt to come into close contact with the body of a wearer owing to the extensibility and contractibility of the side panels. Gaps are hardly produced between the wearer's body and the diaper. This favors leakage prevention but, on the other hand, causes a disadvantage that water vapor generated from the wearer's body tends to remain in the diaper, which can easily result in a rise of humidity and can cause overhydration.

As is described in the publication, the side panels can have air or moisture permeability as well as extensibility. By so designing, a rise in humidity inside the diaper can be suppressed to some extent. Nevertheless, because the part where overhydration occurs most easily is the absorbent member and its vicinities rather than the side panels, endowing the side panels with air or moisture permeability is not enough to sufficiently prevent overhydration. A reduction in absorption capacity of an absorbent member results in a reduction in thickness of the absorbent member, which will bring about improved air permeability of the absorbent member but, in turn, easily result in leakage.

Disclosure of the Invention

The present invention provides an absorbent article according to claim 1.

Brief Description of the Drawings:

Fig. 1 schematically illustrates a cross-section of an embodiment of an absorbent member for an absorbent article according to the present invention.
Fig. 2 is an exploded perspective of the absorbent member shown in Fig. 1.
Fig. 3(a) and Fig. 3(b) schematically illustrate other forms of the absorbent member shown in Fig. 1 (corresponding to Fig. 1).
Fig. 4 schematically illustrates a cross-section of another embodiment of the absorbent member for an absorbent article according to the invention (corresponding to Fig. 1).

Fig. 5 schematically illustrates a cross-section of still another embodiment of the absorbent member for an absorbent article according to the invention (corresponding to Fig. 1).

Fig. 6 schematically illustrates a cross-section of still another embodiment of the absorbent member for an absorbent article according to the invention (corresponding to Fig. 1).

Fig. 7 schematically illustrates a cross-section of still another embodiment of the absorbent member for an absorbent article according to the invention (corresponding to Fig. 1).

Fig. 8 schematically illustrates a cross-section of still another embodiment of the absorbent member for an absorbent article according to the invention (corresponding to Fig. 1).

Fig. 9(a), Fig. 9(b), and Fig. 9(c) each represent another embodiment of the upper absorbent submember of the absorbent member shown in Figs. 4 and 5 and of the absorbent submember of the absorbent member shown in Fig. 6.

Fig. 10(a), Fig. 10(b), and Fig. 10(c) are each an exploded perspective of another embodiment of the absorbent member for an absorbent article according to the invention (corresponding to Fig. 2).

Fig. 11(a) and Fig. 11(b) are each an exploded perspective of still another embodiment of the absorbent member for an absorbent article according to the invention (corresponding to Fig. 2).

Fig. 12 is an exploded perspective of still another embodiment of the absorbent member for an absorbent article according to the invention (corresponding to Fig. 2).

Fig. 13(a), Fig. 13(b), Fig. 13(c), Fig. 13(d), and Fig. 13(e) each schematically illustrate the shape of the fiber accumulated layer of the absorbent member for an absorbent article according to the invention.

Fig. 14(a) and Fig. 14(b) are each a cross-section of still another embodiment of the absorbent member for an absorbent article according to the present invention (corresponding to Fig. 1).

Fig. 15(a), Fig. 15(b), and Fig. 15(c) are each a cross-section of still another embodiment of the absorbent member for an absorbent article according to the present invention (corresponding to Fig. 1).

Fig. 16 schematically illustrates still another embodiment of the absorbent member for an absorbent article according to the invention.

Fig. 17 illustrates the mechanism of liquid absorption by the absorbent member shown in Fig. 16.

Fig. 18 schematically illustrates apparatus that is preferably used to make the absorbent member of Fig. 16.

Fig. 19 schematically illustrates still another embodiment of the absorbent member for an absorbent article according to the invention (corresponding to Fig. 16).

Fig. 20 is a perspective of a disposable diaper as an embodiment of the absorbent article according to the present invention.

Fig. 21 is a cross-section of Fig. 20 taken along line II-II.

Fig. 22 is a schematic fragmentary cross-section taken across the crotch portion of a modification of the disposable diaper shown in Fig. 20.

Fig. 23 is a schematic fragmentary cross-section taken across the crotch portion of another modification of the disposable diaper shown in Fig. 20.

Fig. 24 illustrates body measurements needed in the novel diaper design approach.

Fig. 25 represents a novel diaper design approach, in which Fig. 25(a) illustrates a base pattern obtained by draping, Fig. 25(b) illustrates a corrected base pattern obtained by adding correction to the base pattern, Fig. 25(c) is a revised master pattern obtained by adding correction to a mater pattern taking extension and contraction of the wearer's skin into consideration, and Fig. 25(d) is a final pattern obtained by adding correction to the revised master pattern taking ease of diapering into consideration.

Fig. 26 is a plan of still another modification of the disposable diaper of Fig. 20 in its flat-out, uncontracted state.

Detailed Description of the Invention:

The present invention will be described based on its preferred embodiments with reference to the accompanying drawings. The absorbent article of the present invention is used primarily for absorbing and retaining discharged body fluids such as urine and menstrual blood. The absorbent article of the invention includes disposable diapers, sanitary napkins, and incontinence pads, but is not limited thereto, and a wide range of articles usable to absorb fluids discharged from a human body are included.

The absorbent article of the present invention typically has a topsheet, a backsheet, and a liquid retentive absorbent member interposed between the two sheets. The topsheet and backsheet can be of any material commonly employed in the art with no particular restriction. For example, useful topsheets are liquid permeable sheets made of hydrophilized nonwoven fabrics or perforated films. Useful backsheets are liquid impermeable or water repellent sheets such as a thermoplastic resin film and a laminate of the film and nonwoven fabric. The backsheet may have water vapor permeability. The absorbent article can have other members configured for specific purposes of specific articles. Such other members are well known to those skilled in the art. For example, an absorbent article for application to a disposable diaper or a sanitary napkin may have one or more pairs of standing guards on both lateral sides of the topsheet.

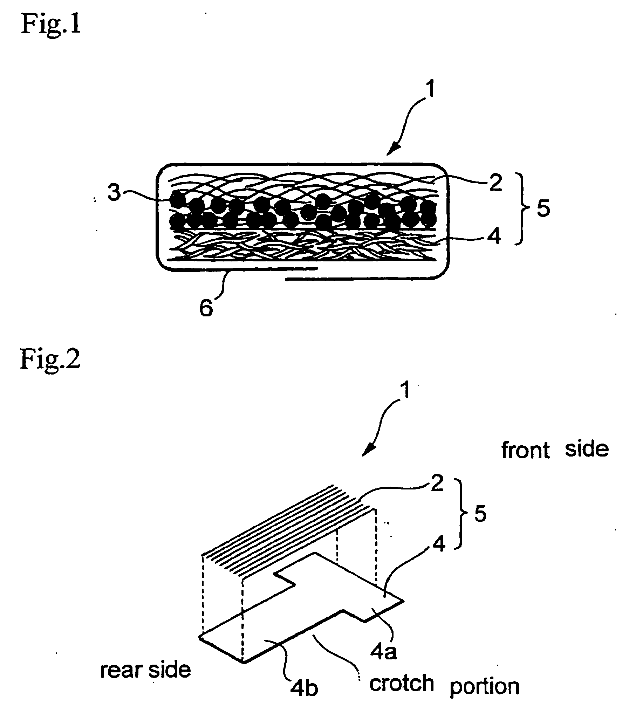

Fig. 1 is a schematic illustration of an embodiment of an absorbent member according to the present invention. The absorbent member 1 of this embodiment is characterized in that it has sufficient absorption capacity and yet is thin and light weight. The thus characterized absorbent member 1 is an absorbent core 5 enclosed in a sheet of a fibrous material (hereinafter referred to as a fiber sheet) 6. The absorbent core 5 has a continuous fiber web (hereinafter simply referred to as a web) 2 containing a superabsorbent polymer 3 and a fiber accumulated layer or nonwoven fabric (hereinafter inclusively referred to as a fiber accumulated layer) 4. In Fig. 1, the upper side is a side to face the skin of a wearer, and the lower side is a side to face the backsheet.

Fig. 2 is a schematic, exploded perspective of the absorbent member 1 shown in Fig. 1. Fig. 1 is a cross-section taken across the longitudinally middle part of the absorbent member 1 of Fig. 2. The fiber sheet 6 is not depicted in Fig. 2. The continuous fiber web 2 has a rectangular shape with its length extending in the longitudinal direction of the absorbent member 1 as illustrated in Fig. 2. The fiber accumulated layer 4 is T-shaped with its length coinciding with the length of the web 2. The T-shaped fiber accumulated layer 4 is assembled into an absorbent article such that the horizontal part 4a thereof is located in the front portion of an absorbent article, i.e., the stomach side of a wearer. The rectangular web 2 is disposed on the upper side of the vertical part 4b of the T-shaped fiber accumulated layer 4 with its width being the same as the width of the vertical part 4b.

The continuous fibers constituting the web 2 are hydrophilic. Hydrophilic continuous fibers that can be used in the present invention include those which are essentially hydrophilic and those which are not essentially hydrophilic but rendered hydrophilic through a hydrophilization treatment. Those which are not essentially hydrophilic but rendered hydrophilic by a hydrophilization treatment include various synthetic fibers having been surface-treated with a surface active agent. The synthetic fibers include single-component fibers made of polyethylene, polypropylene, polyethylene terephthalate, etc. and conjugate fibers containing two or more of these resins. Essentially hydrophilic continuous fibers are preferred. Cellulose acetate or rayon continuous fibers are more preferred. Cellulose acetate continuous fibers are particularly preferred for bulk retention even when wetted. Cellulose acetate is preferably cellulose triacetate or cellulose diacetate. Nylon and acrylic fiber are also usable as continuous fibers to make up the web 2.

In order for the absorbent member 1 to secure liquid permeability, it is desirable for the hydrophilic continuous fibers (and also staple fibers cut from continuous fibers) to have a water content of less than 10% by weight, preferably 1% to 8% by weight. The above water content is preferred, in other words, so as to prevent the fibers from being plasticized and softened on absorbing water and from swelling to clog the absorbent member 1. If fibers with too high a water content are used, the absorbent article can get hard when the fibers are compressed to have a controlled thickness in the manufacture of an absorbent article or when the absorbent article is compressed for a long time, for example, in a package. A hardened absorbent article has reduced wearing comfort and can cause skin troubles due to chafing. The reason for the problem is that a hydrogen bond is formed between different fibers or between different sites of a fiber due to the fibers' absorbing moisture as well as the strong hydrophilicity of the fibers per se. The water content is measured by the method taught in JP-A-7-24003, para. [0025].

The continuous fibers are crimped fibers having a crimp percentage (specified in JIS L0208) of preferably 10% to 90%, more preferably 10% to 60%, even more preferably 20% to 50%. By making a web from crimped continuous fibers, it is easy to embeddedly support a large quantity of a superabsorbent polymer in the web stably, and the superabsorbent polymer, even when used in a large amount, is suppressed from moving extremely or falling off. The means for crimping the continuous fibers is not particularly limited. The crimp may be either two-dimensional or three-dimensional. The percentage of crimp (or crimp percentage) is defined to be a percentage of a difference between the length A of a crimped fiber in its straightened state and the natural length B of the crimped fiber to the length A, being calculated from equation:

$$\text{Percentage of crimp (\%)} = ((A-B)/A) \times 100$$

The natural length of a crimped fiber is the length of the straight line connecting the two ends of a continuous fiber in its natural state. The term "natural state" means a state of a continuous fiber hanging under its own weight with its one end fixed to a horizontal plate. The term "straightened state" means a state of a continuous fiber stretched out until no crimp remains under a minimum load.

The number of crimps of the continuous fibers having the recited percentage of crimp is preferably 2 to 25, more preferably 4 to 20, even more preferably 10 to 20, per centimeter.

The thickness of the continuous fibers is not particularly limited. Satisfactory results can generally be obtained by using continuous fibers having a thickness of 1.0 to 7.8 dtex, preferably 1.7 to 5.6 dtex. The terminology "continuous fiber" as used throughout the description means a fiber having a fiber length preferably of 70 mm or longer, more preferably 80 mm or longer, even more preferably 100 mm or longer, as measured by the mean fiber length measurement method (method C) specified in JIS L 1015. In cases where the length of a web per se is shorter than 100 mm, "continuous fiber" is defined as follows. When preferably at least 50%, more preferably 70% or more, even more preferably 80% or more, of the fibers making up the web extend over the whole length of the web, the fibers of the web are regarded as continuous fibers. The continuous fibers used in the present invention are those generally termed "continuous filaments". A bundle of continuous filaments is generally termed "a tow". Accordingly, the terminology "continuous fiber" as used herein shall include a continuous filament. The expression "a web having continuous fibers oriented" or its equivalent shall include a bundle of continuous fibers, namely, a tow, as a raw material for making a web and a tow layer of continuous filaments.

The superabsorbent polymer is embeddedly supported in the web. The phrase "embeddedly supported" is intended to indicate the state in which the superabsorbent polymer enters the interfiber spaces formed by the crimped continuous fibers and therefore hardly moves extremely or falls off with the wearer's movement. This state is the results of the continuous fibers' entangling with or catching on the superabsorbent polymer or the polymer's attaching to the continuous fibers by its own stickiness. Because the spaces among the continuous fibers are easily deformable when externally stressed and also because the stress is absorbed by the whole continuous fibers, the spaces are protected from destruction. The superabsorbent polymer has at least part thereof embedded in the web 2. Under some conditions of making the absorbent member 1, almost the whole of the superabsorbent polymer is uniformly embedded into the web 2.

The superabsorbent polymer 3 is usually particulate. A fibrous superabsorbent polymer is also useful. A particulate superabsorbent polymer which is irregular in shape or blocky or rodlike can be embeddedly supported in the web in an amount one to ten times the weight of the web. A particulate superabsorbent polymer which is spherical particles or agglomerates of spherical particles can be embeddedly supported by the web in an amount one to five times the weight of the web. Where both high absorption and thinness are demanded, the former type of polymers is preferably used. Where weight is put on hand (reduction of crispness characteristic of a superabsorbent polymer), it is desirable to use the latter type of the polymers. The superabsorbent polymer 3 is embeddedly supported in the web 2. In Fig. 1, the superabsorbent polymer 3 is localized from the middle of the thickness to the lower part of the web 2. The superabsorbent polymer has part thereof embedded in the web 2. Under some conditions of making the absorbent member 1, almost the whole of the superabsorbent polymer 3 is uniformly embedded into the web 2. The term "uniform" or "uniformly" is used not only when the superabsorbent polymer is distributed completely uniformly in the thickness or width direction of the absorbent member 1 but also when the variation in weight of the polymer per unit area among different parts of the absorbent member 1 is such that the maximum is within double the minimum. Such variation is attributed to a phenomenon infrequently faced in the manufacture of an absorbent article that the superabsorbent polymer is fed in excess and sprinkled in a part in an extremely large amount. That is, under the term "uniform" is included an unavoidable nonuniform distribution, but an intentional variation in amount of the superabsorbent polymer is not included under the term.

Where the superabsorbent polymer 3 is nonuniformly distributed in the thickness direction as in Fig. 1, the absorbent member exhibits high liquid spreading ability and high absorbing ability and is therefore fit for use in a disposable diaper. Where, on the other hand, the superabsorbent polymer 3 is localized from the middle of the thickness to the upper part of the web 2, the absorbent member exhibits high spot absorptivity and is therefore suited for use in a mild incontinence pad or a sanitary napkin.

Since the continuous fibers have crimp as stated, they furnish a great number of spaces capable of retaining particles, in which spaces the superabsorbent polymer is retained. Therefore, a large quantity of the superabsorbent polymer sprinkled on the web hardly moves extremely or falls off. Furthermore the structure of the absorbent member 1 is hardly destroyed by active movement of a wearer. The percentage of crimp and the amount of the continuous fibers are selected appropriately depending on the kind of the superabsorbent polymer used. A conventional absorbent member could retain a large quantity of a superabsorbent polymer by increasing the amount of a fibrous material, which results in increases in weight and thickness of the absorbent member. In contrast, the present invention makes it possible to increase the amount of the superabsorbent polymer relative to the amount of the fibrous material. Specifically, the absorbent member 1 preferably contains the superabsorbent polymer in an amount equal to or larger than, more preferably twice or more as much as, even more preferably three times or more as much as, the weight of the continuous fibers. Such high polymer retainability has accomplished reduction in thickness and weight of the absorbent member 1. The maximum weight per unit area of the superabsorbent polymer retainable by the continuous fibers is decided in connection with prevention of extreme movement or fall-off of the polymer. While depending on the degree of crimping of the continuous fibers, the superabsorbent polymer hardly moves extremely or falls off against active wearer's movement as long as the weight of the superabsorbent polymer is not more than about ten times the weight of the continuous fibers.

A "supporting ratio" determined as follows can be adopted as a measure for evaluating how stably a superabsorbent polymer is embeddedly supported. Continuous fibers are made into a web with a uniform thickness measuring 100 mm in width and 200 mm in length. The amount of the continuous fibers is adjusted to give a web weighing 26 $g/m^2$. The web is stretched in the fiber orientation direction to adjust the percentage of crimp. Since the weight per unit area of the web changes from 26 $g/m^2$ due to the stretch, the web is weighed to calculate an accurate weight per unit area.

A polymer is sprinkled onto the web while maintaining the adjusted crimp percentage. The web is trimmed into a 100 mm by 200 mm web. Sprinkling the polymer is performed as follows. The web is transversely placed and strewed evenly with the polymer by the hand to give a weight per unit area of the polymer 10 times the weight per unit area of the web. After completion of the polymer sprinkling, the web is wholly wrapped in tissue paper having a grammage of 16 g/m$^2$ and then released from the stretch. The tissue paper and the web are united with an adhesive.

A 100 mm x 100 mm specimen is cut out from the middle part of the web. In case where the tissue paper is joined to itself on being cut to close the cut end, it is separated apart from itself. The cut-out specimen is hang with the continuous fibers vertical and shaken horizontally at an amplitude of 5 cm at a rate of one shake per second. The polymer fallen from the cut end is weighed. The weight is taken as S1. A supporting ratio is calculated according to equation below from the value S 1 and the weight S0 of the superabsorbent polymer having been contained in the specimen before the shaking (i.e., 10 times the weight of the web).

$$\text{Supporting ratio (\%)} = \{1-(S1/S0)\} \times 100$$

When the thus calculated supporting ratio is 60% or more, preferably 70% or more, more preferably 80% or more, it is safe to say that the superabsorbent polymer hardly falls off the web.

A "moving ratio" determined as follows can also be used in addition to the aforementioned supporting ratio as a measure for evaluating how stably a superabsorbent polymer is embeddedly supported. The 100 mm x 100 mm specimen used in the supporting ratio measurement is weighed beforehand to obtain the initial weight W0. After the supporting ratio measurement, the specimen is cut into equal halves across the extending direction of the continuous fibers. Each of the halves is weighed. The weight W1 of the one that shows a larger change from half the initial weight W0 of the specimen than the other is used to calculate the moving ratio. That is, when the weights of the two halves, taken as W1' and W1", satisfy the relationship: |W1'-w0/2|>|W1"-W0/2|, W1=W1'.

The moving ratio is calculated according to equation below from the thus decided W1 value and the initial weight W0 of the specimen.

$$\text{Moving ratio (\%)} = \{1-W1/(W0/2)\} \times 100$$

When the thus determined moving ratio is within 40%, preferably 30% or less, more preferably 20% or less, it is safe to say that the superabsorbent polymer hardly moves in the web.

The following method of evaluation using the specimen that has been subjected to the above-described supporting ratio measurement is also useful. Fifty grams of physiological saline (0.9 wt% NaCl) is evenly applied onto the specimen that has been subjected to the supporting ratio measurement to observe the swell. When the variation in thickness of the swollen specimen is such that the maximum is not larger than double the minimum, it is safe to say that the polymer hardly moves extremely or falls off.

The specimen to be used in the above-described methods of evaluation should be cut out of an area where the superabsorbent polymer is sprinkled evenly in the planar direction of the web.

In case where the web has insufficient ability to embeddedly support the superabsorbent polymer, it is possible to add into the web a hot-melt adhesive, a binder of various kinds (e.g., acrylic emulsion pressure-sensitive adhesive), sugar derivatives (e.g., carboxymethyl cellulose and ethyl cellulose) or a thermoplastic resin (e.g., polyethylene or polypropylene) as appropriate. The web may be embossed, or a flock-finished sheet may be used in combination with the web. Where the continuous fibers are soluble or plasticizable in a solvent, an appropriate solvent may be used in combination. For example, triacetin may be added to continuous fibers containing cellulose acetate to form bonds in part of the web. The bonds not only improve the superabsorbent polymer supporting properties of the web but also make the web stiff and resistant to twisting or deformation as long as the amount of the solvent to be added and the method of treatment are properly selected. The web with moderate stiffness makes the absorbent article easier for a user to handle and prevents the absorbent article from gathering too much or losing its shape.

In the absorbent member 1 of the present embodiment, the continuous fibers making up the web 2 are unidirectionally oriented in the planar direction of the absorbent member 1. Owing to the unidirectional fiber orientation, liquid absorbed by the absorbent member 1 diffuses preferentially in the orientation direction of the continuous fibers, i.e., in the planar direction of the absorbent member 1. Liquid diffusion in the direction perpendicular to the orientation direction of the continuous fibers is suppressed. When the continuous fibers are oriented in the length direction of the absorbent article, side leakage (leakage from both sides of the absorbent article) is effectively prevented.

The degree of orientation of the continuous fibers is preferably 1.2 or higher, more preferably 1.4 or higher, as measured with a microwave molecular orientation analyzer MOA-2001 A manufactured by Kanzaki Co., Ltd. Measurement is made on three points of a specimen measuring 50 mm in width and 100 mm in length to obtain an average. Where a sample is smaller than that size, two or more samples are placed adjacent to each other without overlap.

Where the continuous fibers are oriented in the length direction of an absorbent article, it is preferred that there be no linear joint line across the orientation direction of the continuous fibers in the absorbent member. Such a joint line would block smooth fluid distribution in the orientation direction, which can cause side leaks.

Where the continuous fibers are oriented in the width direction of an absorbent article, spot absorptivity is obtained with longitudinal fluid distribution suppressed. In this case, it is preferred that the absorbent member have a joint line across the orientation direction of the continuous fibers to prevent side leakage. The term "line" in "joint line" means a continuous line capable of inhibiting penetration of a liquid. This does not mean that the individual sealing lines, etc. must be linked to one another continuously. For instance, when sealing lines formed spacedly but with overlaps when seen from a certain direction are capable of hindering migration of a liquid in that direction, the sealing lines can be said to be a joint line. The joint line may be curved or piecewise straight as well as straight linear. The joint line width is preferably about 0.2 to 15 mm.

A joint line may be formed only in the web 2 or through the whole thickness of the absorbent member 1 including the fiber sheet 6. A linear joint may be such that the topsheet is joined to the absorbent member. In any configuration, a joint line is preferably formed in the longitudinally middle portion of the absorbent article. A joint line may be formed outboard of both lateral side edges of the absorbent member. If a liquid moves in the web through capillarity, it will run into the joint line and is blocked from moving farther thereby prevented from causing side leakage.

The absorbent member 1 of the present embodiment has the fiber accumulated layer 4 superposed on the lower side of the web 2. The fiber accumulated layer 4 may or may not contain a superabsorbent polymer. The fiber accumulated layer 4 is obtained by accumulating natural pulp and/or synthetic fibers, or the fiber accumulated layer 4 is a nonwoven fabric made of natural pulp and/or synthetic fibers. The fiber accumulated layer 4 obtained by accumulating natural pulp and/or synthetic fibers may be any of fiber accumulated layers that have been commonly used as an absorbent member in conventional absorbent articles. The nonwoven fabric made of natural pulp and/or synthetic fibers that can be used as the fiber accumulated layer 4 includes airlaid nonwoven fabric. Provided underneath the web 2, the fiber accumulated layer 4 serves as a temporary liquid reservoir. As a result, the absorbent member 1 effectively prevents leakage even when a body fluid is discharged at a high speed as in urination. To ensure the effect, it is preferred for the superabsorbent polymer embeddedly supported in the web 2 to be localized in the backsheet facing side of the web 2 as is shown in Fig. 1. When the superabsorbent polymer is incorporated into not only the web 2 but the fiber accumulated layer 4, the anti-leakage effect is further ensured. Moreover, the superabsorbent polymer in the web 2 acts as a moisture absorbent thereby to control a humidity rise inside the absorbent article while worn. As a result, the absorbent article hardly causes stuffiness.

The natural pulp as a fiber of the fiber accumulated layer 4 includes wood pulp. The synthetic fiber as a fiber of the fiber accumulated layer 4 includes those called staple fibers preferably with an average length of 0.1 to 30 mm, more preferably 0.5 to 25 mm, even more preferably 1.5 to 15 mm. The fineness of the synthetic fibers is preferably 0.1 to 7.8 dtex, more preferably 0.5 to 5.6 dtex, even more preferably 0.9 to 3.4 dtex. Useful synthetic fibers include single-component fibers made of polyethylene, polypropylene, polyethylene terephthalate, etc. and conjugate fibers made of two or more of these resins. Conjugate fibers are preferred in view of ease of providing fusion bondability between individual fibers as well as various known functions and ease of making the fiber accumulated layer bulky. The conjugate fibers include sheath-core or side-by-side bicomponent fibers. The synthetic fibers may have a circular cross-section or a modified cross-section, such as a C-shaped cross-section or a hollow cross-section. The single-component fibers preferably have a modified cross-section.

Synthetic pulp can be used as a synthetic fiber. Exemplary synthetic pulp includes those based on a thermoplastic resin such as polyethylene, modified polyethylene or polypropylene. It is preferred for the synthetic pulp to have an average fiber length of 0.1 to 10 mm, more preferably 0.5 to 5 mm, even more preferably 0.9 to 1.5 mm, so that it may be handled in the same way as for natural pulp such as wood pulp.

Use of natural pulp improves absorption capacity of the absorbent member 1 because natural pulp is, in general, a material having high liquid absorptivity and high liquid distribution ability. On the other hand, because synthetic fiber has properties of melting on being heated to a given temperature, the fiber accumulated layer 4 containing synthetic fiber or the whole absorbent member 1 may be heated to melt the synthetic fiber thereby to increase the strength of the absorbent member as a whole. When in using natural pulp as a fiber, the fiber accumulated layer 4 is in most cases a layer obtained by accumulating natural pulp. When in using synthetic fiber as a fiber, the fiber accumulated layer 4 is in most cases a nonwoven fabric such as an airlaid nonwoven fabric.

The fiber accumulated layer 4 may contain, in addition to the above-described fibers, natural or (semi)synthetic hydrophilic staple fibers of rayon, cotton, Lyocell, Tencel, cellulose acetate, polyvinyl alcohol fiber, acrylic fiber, and so forth.

As shown in Fig. 1, the absorbent core 5 composed of the web 2 and the fiber accumulated layer 4 is entirely wrapped in the fiber sheet 6. In the present embodiment, the fiber sheet 6 is a single sheet. The fiber sheet 6 covers the upper surface of the absorbent core 5, wraps around both the lateral sides of the absorbent core 5, and meets and overlaps with itself at the widthwise middle of the lower surface of the absorbent core 5.

Wrapping the absorbent core 5 with the fiber sheet 6 effectively prevents the superabsorbent polymer 3 in the core 5 from extremely moving or falling off and improves the handling properties of the absorbent member 1 as an independent unitary member that can easily be transported. Being so wrapped in, the absorbent core can be trimmed or cut out with ease to provide an absorbent member of any desired shape according to the shape of an absorbent article.

When the absorbent core 5 and the fiber sheet 6 are joined by a prescribed means, the absorbent member 1 will exhibit increased stiffness by the joint between the core 5 and the sheet 6 and by the stiffness possessed by the sheet 6, which further improves the handling properties of the absorbent member 1. The means for joining the absorbent core 5 and the fiber sheet 6 include adhesive application and heat fusion.

The fiber sheet 6 is of material having strength enough to prevent fall-off of the superabsorbent polymer and causing no hindrance to permeation of a discharged fluid. For example, tissue paper or liquid permeable nonwoven fabric can be used. The nonwoven fabric may be hydrophilized or perforated if needed. Slits may be cut in the nonwoven fabric. The fiber sheet 6 may be made softer by embossing. The nonwoven fabrics that can be used as the fiber sheet 6 include those made of single-component fibers of thermoplastic resins, such as polyethylene, polypropylene, and polyethylene terephthalate, and those made of conjugate fibers containing two or more of these resins. Examples of the nonwoven fabrics are thermal-bonded nonwoven fabrics, spun-bonded nonwoven fabrics, spunbonded-meltblown-spunbonded nonwoven fabrics, spunbonded-meltblown-meltblown-spunbonded nonwoven fabrics, needle punched nonwoven fabrics, hydroentangled nonwoven fabrics, and airlaid nonwoven fabrics. These nonwoven fabrics may contain hydrophilic fibers, such as rayon, cotton, Lyocell, Tencel, cellulose acetate, and natural pulp.

A preferred process of producing the absorbent member 1 of the present embodiment will be described. First of all, a tow of crimped continuous fibers is prepared. The tow is conveyed while being stretched under tension in the machine direction. In this state, the tow is opened into a web. The step of opening the tow is achieved using, e.g., an air opening apparatus utilizing compressed air. Having crimp, the continuous fibers easily stretch under tension in the machine direction. A superabsorbent polymer is sprinkled on the stretched web. Before sprinkling, the running speed of the web is slowed down, and the web is transferred onto a vacuum conveyor. The web on the vacuum conveyor is released from the tension. On release from tension, the continuous fibers return to their crimped state. At this time, the continuous fibers in the web have the above-recited percentage of crimp. In addition, the web is bulkier than it has been under tension. As a result, the web has gained improved ability to retain a superabsorbent polymer. In this state, a superabsorbent polymer is sprinkled. The crimped continuous fibers have interfiber spaces in which the superabsorbent polymer can be embedded and retained. In this fashion, a desired amount of the superabsorbent polymer can be embeddedly supported in the web. In contrast, it is difficult to successfully embed and retain the superabsorbent polymer in the web in the stretched state under tension; for the stretched web is incapable of providing ample space between fibers enough to hold the polymer. It is effective in helping the polymer be embeddedly retained to suck the web from the reverse side simultaneously with the polymer sprinkling. It is possible to vary the polymer distribution in the web thickness direction by appropriately adjusting the degree of suction.

The above-described air opening apparatus may be replaced with, or used in combination with, a roll having a number of discs spacedly arranged around its periphery along the axial direction and a smooth backup roll, between which a tow is passed and opened. In this case, there is produced unevenness in amount or density of the continuous fibers, resulting in formation of a web having an uneven structure with projections and depressions. The superabsorbent polymer is then sprinkled on the web having such an uneven structure. Part of the sprinkled superabsorbent polymer drops into the depressions. On narrowing the web, the superabsorbent polymer is shut in between adjacent projections and thereby firmly embedded in the web.

Prior to the step of sprinkling the superabsorbent polymer, an adhesive such as a hot-melt adhesive is applied to the web. The step of applying the adhesive is carried out either by a contact application method, such as roll coating or screen printing, or a non-contact application method, such as spray coating. Spray coating, a non-contact application method, is preferred for easy changeover of application patterns and adjustability of the amount of the adhesive to be applied. In particular, a spray coating system fit for bonding in points is preferred. Spray coating systems include slot spraying, curtain spraying, melt blown spraying, and spiral spraying. The amount of the adhesive to be applied is preferably as small as not to become a hindrance to migration of liquid. From this viewpoint, the amount of the adhesive to be applied preferably ranges from 3 to 30 $g/m^2$, more preferably 5 to 15 $g/m^2$.

After the adhesive is applied, the superabsorbent polymer is sprinkled on the web and retained in the spaces formed by the crimped continuous fibers. After the polymer sprinkling, the web may be compressed by means of a roll, a belt, etc. to embed the superabsorbent polymer in the web more stably. The superabsorbent polymer is thus embeddedly supported in the web.

The resulting web and a fiber accumulated layer separately prepared using an airlaying apparatus are superposed on each other to obtain an absorbent core. Separately from the operation of superposition, an adhesive such as a hot melt adhesive is applied discontinuously on a side of a fiber sheet. The absorbent core is placed on the adhesive-applied side of the fiber sheet and wrapped in the fiber sheet to make the absorbent member 1.

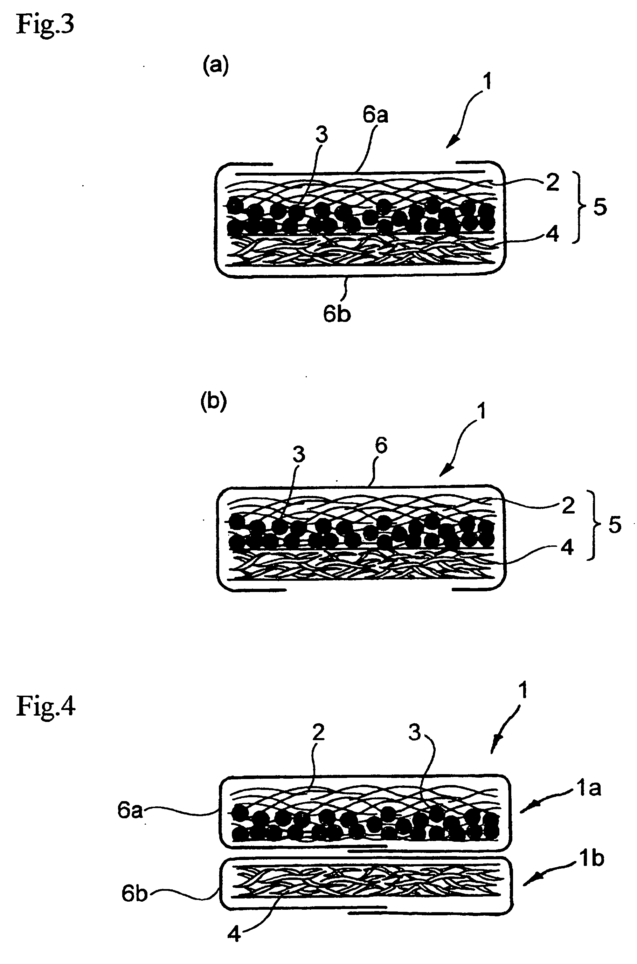

The manner of wrapping the absorbent core 5 with the fiber sheet 6 is not limited to the illustration of Fig. 1. For instance, the absorbent core 5 may be wrapped in fashions illustrated in Figs. 3(a) and 3(b). The absorbent member 1 of Fig. 3(a) has the absorbent core 5 covered with a first fiber sheet 6a on its upper side and with a second fiber sheet 6b on its lower side and lateral sides. The second fiber sheet 6b wraps around the sides to the upper side of the absorbent core 5 and overlaps with both side margins of the first fiber sheet 6a. The absorbent member 1 of Fig. 3(b) has the absorbent core 5 covered with a single fiber sheet 6 similarly to the absorbent member 1 of Fig. 1. While, in Fig. 1, the opposing lateral sides of the fiber sheet 6 overlap with each other on the lower side of the absorbent core 5, the opposing lateral sides of the fiber sheet 6 of Fig. 3(b) cover only the lateral side portions of the lower side of the absorbent core 5, with the laterally middle portion of the lower side left uncovered.

In applications to disposable diapers for babies, the absorbent member 1 is of thin type, preferably having a thickness of 1 to 4 mm, more preferably 1.5 to 3 mm, irrespective of whichever of the above configurations it may have. In applications to sanitary napkins, the thickness is preferably 0.5 to 3 mm, more preferably 1 to 2 mm. For applications to incontinence pads, the thickness is preferably 0.5 to 4 mm, more preferably 1 to 3 mm.

The thickness of the absorbent member 1 is measured with a load of 0.245 kPa applied thereto by placing a 5 cm square acrylic resin plate and a weight. In the present embodiment, the thickness was measured with a laser displacement sensor LK080 class 2 available from Keyence Corp. Measurement is made at five positions to obtain an average. If a piece of data fluctuates 20% or more, the piece is tossed out and replaced with an additional one. Prior to the measurement, a load of 24.5 kPa is applied to the sample for 12 hours to straighten wrinkles.

In order to make the absorbent member 1 with the above-recited thickness in the present embodiment, it is preferred for the web 2 to have a weight of 120 to 400 $g/m^2$, more preferably 150 to 300 $g/m^2$, for applications to disposable diapers for babies; 35 to 200 $g/m^2$, more preferably 50 to 150 $g/m^2$, for applications to sanitary napkins, or 35 to 500 $g/m^2$, more preferably 50 to 400 $g/m^2$, for applications to incontinence pads.

From the same standpoint, the fiber accumulated layer 4 of the absorbent member 1 according to the present embodiment preferably has a weight of 20 to 300 $g/m^2$, more preferably 50 to 200 $g/m^2$ for applications to disposable diapers for babies; 20 to 500 $g/m^2$, more preferably 50 to 300 $g/m^2$, for applications to sanitary napkins, or 20 to 500 $g/m^2$, more preferably 50 to 300 $g/m^2$, for applications to incontinence pads; whether or not the fiber accumulated layer 4 contains the superabsorbent polymer.

Compared with the weights of the web 2 and the fiber accumulated layer 4, the weight of the fiber sheet 6 is not so critical in the present invention. It suffices for the fiber sheet 6 to have a weight of about 5 to 80 $g/m^2$, preferably about 10 to 50 $g/m^2$.

Other embodiments of the present invention will then be described with reference to Figs. 4 through 15. The description of the above embodiment applies appropriately to those particulars of the other embodiments that are not described here. Elements in Figs. 4 to 15 identical to those in Figs. 1 to 3 are given the same numerals as in Figs. 1 to 3.

The absorbent member 1 shown in Fig. 4 has an upper absorbent submember 1a and a lower absorbent submember 1b. The upper absorbent submember 1a has an absorbent core formed of a web 2 of hydrophilic continuous fibers and a single first fiber sheet 6a wrapping the absorbent core. The web 2 contains a superabsorbent polymer 3. The lower absorbent submember 1b has an absorbent core formed of a fiber accumulated layer 4 and a single second fiber sheet 6b wrapping the absorbent core. The fiber accumulated layer 4 may or may not contain a superabsorbent polymer.

The first and second fiber sheets 6a and 6b may be the same or different in kind and/or weight per unit area.

In the manufacture of an absorbent article, the absorbent member 1 of the present embodiment is made by superposing the separately prepared upper absorbent submember 1a containing the continuous fiber web 2 and lower absorbent submember 1b containing the fiber accumulated layer 4 on each other. This provides an advantage that the production process is free from complexity.

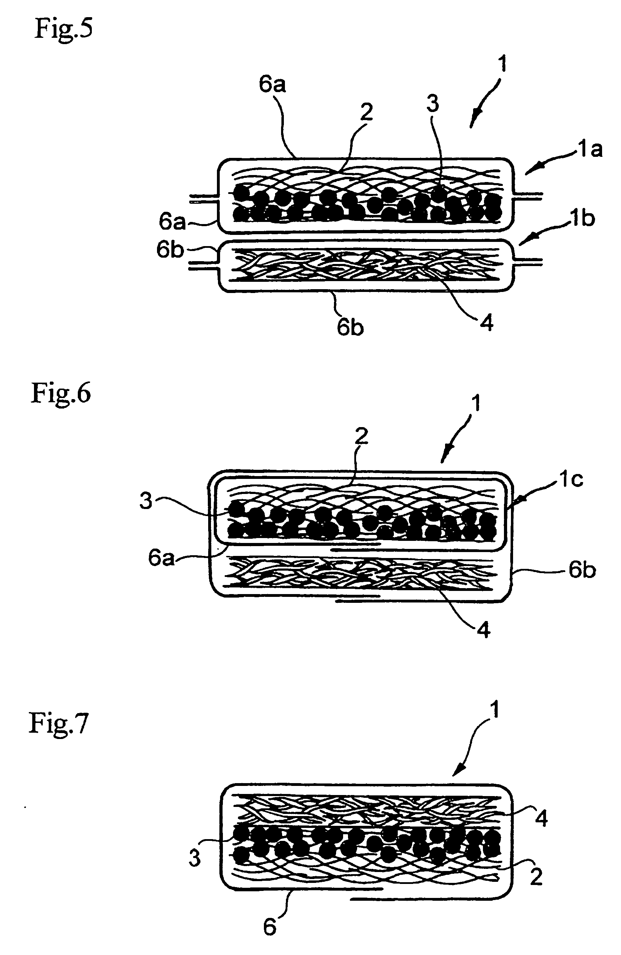

The absorbent member 1 shown in Fig. 5 is similar to that of Fig. 4 in that it has an upper absorbent submember 1a and a lower absorbent submember 1b. The difference is that the upper and lower absorbent submembers 1a and 1b in Fig. 4 are each covered with a single fiber sheet whereas the upper and lower absorbent submembers I a and 1b in Fig. 5 are each covered with two fiber sheets.

Each of the absorbent submembers 1a and 1b is covered on its upper and lower sides with the respective fiber sheets, and the two fiber sheets laterally outwardly extend from both lateral side edges of the absorbent core and are joined together at the extensions. The present embodiment produces the same effects as by the embodiment of Fig. 4. The upper absorbent submember 1a and the lower absorbent submember 1b may have different widths.

The two first fiber sheets 6a constituting the upper absorbent submember 1a may be the same or different in kind and/or weight per unit area. The same applies to the two second fiber sheets 6b constituting the lower absorbent submember 1b.

The absorbent member 1 shown in Fig. 6 has an absorbent submember 1c. The absorbent submember 1c has an absorbent core formed of a hydrophilic continuous fiber web 2 wrapped in a single first fiber sheet 6a. The web 2 contains a superabsorbent polymer. A fiber accumulated layer 4 is superposed on the lower side of the absorbent submember 1c. The fiber accumulated layer 4 may or may not contain a superabsorbent polymer. The absorbent submember 1c and the fiber accumulated layer 4 are wrapped as a unit in a single second fiber sheet 6b. According to this embodiment, since the web 2 containing the superabsorbent polymer 3 is double wrapped in the first fiber sheet 6a and the second fiber sheet 6b, fall-off of the superabsorbent polymer is prevented more effectively.

In the embodiments shown in Figs. 4 through 6, it is desirable that the superabsorbent polymer be localized in the garment facing side with respect to the thickness direction of the web 2. As a result, a continuous fiber-containing web layer is provided in the skin facing side of the absorbent member. This design offers the following advantages. (1) Liquid absorbed by the absorbent member hardly comes back to the surface. (2) Gel blocking of the superabsorbent polymer after absorption is suppressed so that the absorbent member exhibits excellent absorbency for repeated discharge of a body fluid. (3) The surface of the topsheet, the skin facing surface of an absorbent article, has an improved touch.

The continuous fiber web 2 is disposed on the upper side of the fiber accumulated layer 4 in the foregoing embodiments, conversely, the continuous fiber web 2 can be superposed on the lower side of the fiber accumulated layer 4 as illustrated in Fig. 7. The way of superposing the fiber accumulated layer 4 and the continuous fiber web 2 as shown in Fig. 7 is advantageous for improving absorptivity of an absorbent article when a wearer's body pressure imposed thereto, for example, when a wearer is in a lying or sitting posture.

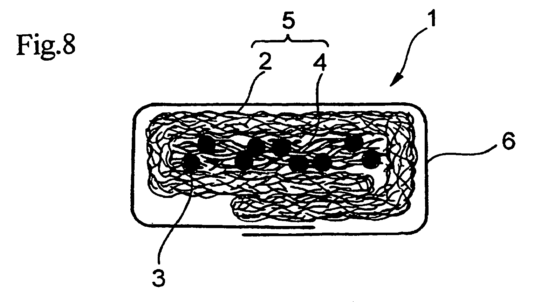

The absorbent member 1 shown in Fig. 8 has an absorbent core 5 composed of a continuous fiber web 2 and a fiber accumulated layer 4. The fiber accumulated layer 4 may or may not contain a superabsorbent polymer 3. The web 2 may or may not contain a superabsorbent polymer 3.

The fiber accumulated layer 4 is wrapped in the web 2, and the web 2 is wrapped in a fiber sheet 6. According to this embodiment, the absorbent member 1 exhibits good recovery from compression, an increased rate of absorbing a discharged liquid, and improved shape retention. These effects are particularly pronounced when in using an airlaid nonwoven fabric as the fiber accumulated layer 4. While airlaid nonwovens generally tend to have a hard texture in the nature of the process of production, the hardness can be reduced by wrapping the airlaid nonwoven fabric with the continuous fiber web 2.

Where an airlaid nonwoven fabric is used as a fiber accumulated layer 4, the nonwoven fabric may be perforated or slitted if desired. The nonwoven fabric may be made softer by embossing.

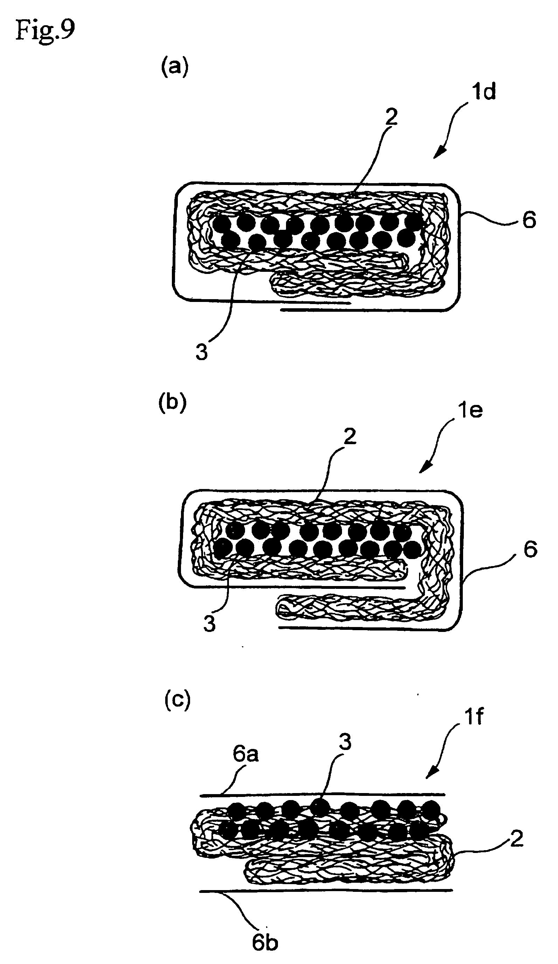

In the absorbent members 1 of the embodiments shown in Figs. 4 and 5, the upper absorbent submember 1a may be replaced with any of absorbent submembers 1d to 1f illustrated in Figs. 9(a) to 9(c). The same replacement is also applicable to the absorbent submember 1c of the absorbent member 1 according to the embodiment of Fig. 6.

The absorbent submember 1d shown in Fig. 9(a) has a continuous fiber web 2 tri-folded inward along its longitudinal direction, a superabsorbent polymer 3 retained in the inside of the tri-folded web 2, and a fiber sheet 6 wrapping the web 2 containing the superabsorbent polymer 3. The absorbent submember 1e shown in Fig. 9(b) has a continuous fiber web 2 and a fiber sheet 6 tri-folded as a unit inward along their longitudinal direction and a superabsorbent polymer 3 retained in the inside of the tri-folded web 2. The absorbent submember 1f shown in Fig. 9(c) has a continuous fiber web 2 folded into three panels in an accordion manner, a superabsorbent polymer 3 embeddedly supported in the panel closer to the upper side of the tri-folded web 2, and two fiber sheets 6a and 6b covering the upper and lower sides of the web 2 containing the superabsorbent polymer 3. The two fiber sheets 6a and 6b may be either the same or different in kind and/or weight per unit area.

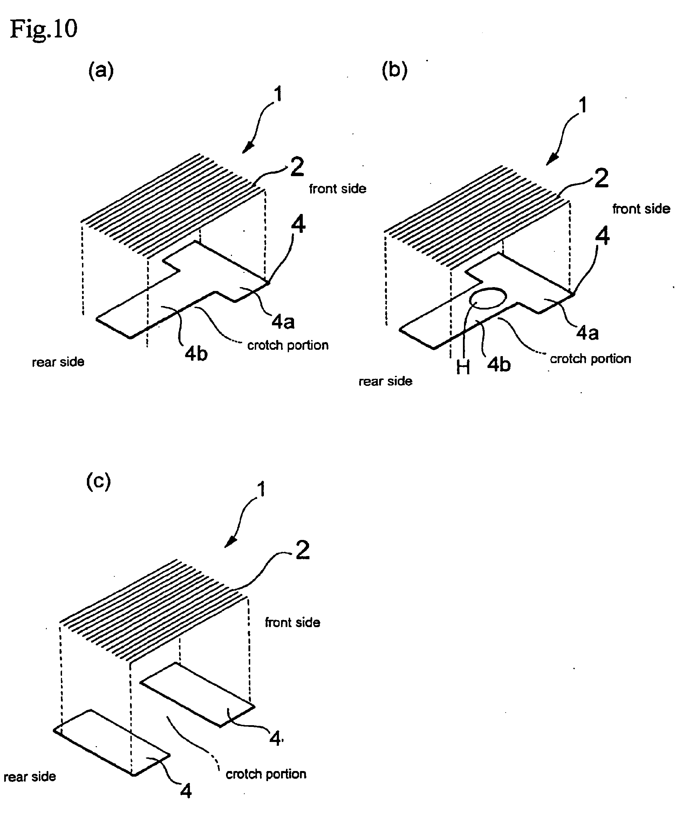

The shapes of the continuous fiber web 2 and the fiber accumulated layer 4 are not limited to those illustrated in Fig. 2. For example, they may have the configurations illustrated in Figs. 10 through 13. The absorbent member 1 of the embodiment shown in Fig. 10(a) has a T-shaped fiber accumulated layer 4 similarly to the absorbent member of the embodiment shown in Fig. 2. The web 2 is disposed on the upper side of the T-shaped fiber accumulated layer 4 with its width being the same as the width of the horizontal part 4a and with its length agreeing with the length of the fiber accumulated layer 4. Fig. 10(b) shows a modification of the absorbent member of Fig. 10(a). The absorbent member 1 of Fig. 10(b) is the same as the one illustrated in Fig. 10(a), except that the vertical part 4b of the T-shaped fiber accumulated layer 4 has a hole H cut out. The cut-out hole H may have a circular shape, an oval shape, a polygonal shape or the like. The purpose of boring the hole H is to make the absorbent member more flexible in the crotch portion of an absorbent article. Existence of a wide absorbent member in the crotch portion of an absorbent article has caused disadvantages such as bunching of the absorbent member during use, resulting in unstable absorbency, lack of fit in the crotch portion, and skin troubles. Although there is fear that mere cutting out a hole H results in reduction of absorbency, a reduction in absorbency can be compensated for by the web 2 that is disposed on the upper or lower side of the fiber accumulated layer 4 to cover the cut-out hole H. While the absorbent article is worn, the cut-out hole H is compressed in the width direction to be closed partly or narrowed and thereby performs the function as a fluid distribution channel.

The absorbent member 1 illustrated in Fig. 10(c) has the fiber accumulated layer 4 provided only in the portions forward and rearward of the crotch portion of an absorbent article but not in the crotch portion. Each piece of the fiber accumulated layer 4 is rectangular. On the other hand, the web 2 is a rectangle disposed on the upper side of the fiber accumulated layer 4 in a continuous area from and including the front portion to and including the rear portion of the absorbent article. The pieces of the fiber accumulated layer 4 and the web 2 all have the same width. While in this embodiment the absorbent member 1 has no fiber accumulated layer 4 at all in the crotch portion, the absorbent member 1 may have the fiber accumulated layer 4 in part of the crotch portion.

The absorbent article having the absorbent member illustrated in Figs. 10(a) through 10(c) has improved flexibility in its crotch portion to provide a good fit to the wearer's body, and the good fit will secure absorbency.

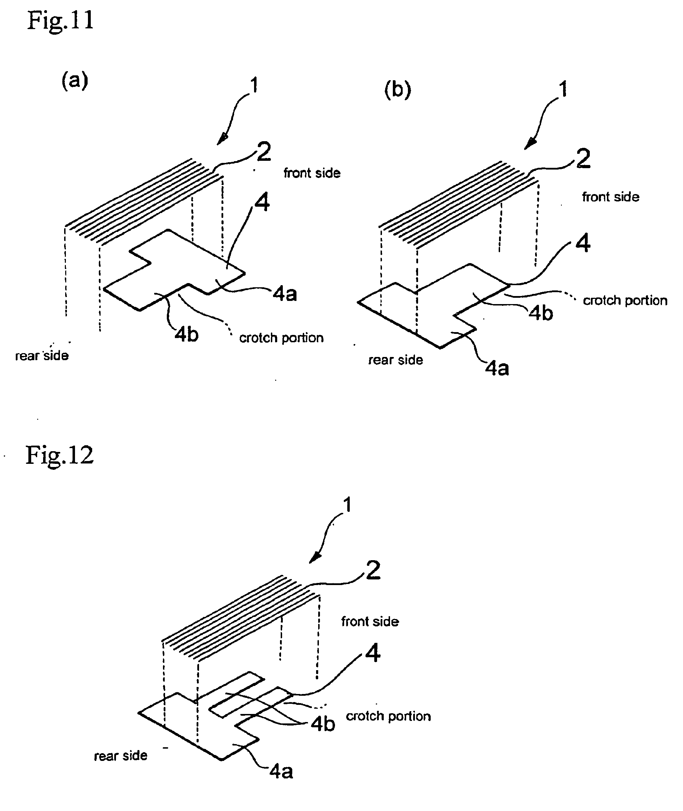

The absorbent member 1 illustrated in Fig. 11(a) has a T-shaped fiber accumulated layer 4. The horizontal part 4a of the T-shape of the fiber accumulated layer 4 is located in the front portion of an absorbent article, and the vertical part 4b of the T-shape is located in an area from and including the front portion to and including the crotch portion. The vertical part 4b does not extend to the rear portion. The web 2 is a rectangle disposed on the upper side of the vertical part 4b of the T-shape of the fiber accumulated layer 4 over the area from and including the front portion to and including the rear portion with its width equal to that of the vertical part 4b.

The absorbent member 1 illustrated in Fig. 11(b) has a T-shaped fiber accumulated layer 4 similarly to the embodiment of Fig. 11(a). The difference from the embodiment of Fig. 11(a) resides in that the horizontal part 4a of the T-shape of the fiber accumulated layer 4 goes in the rear portion of an absorbent article and that the vertical part 4b of the T-shape extends from and including the rear portion to and including the crotch portion of the absorbent article. There is no vertical part 4b in the front portion. A web 2 is arranged in the same configuration as in the embodiment of Fig. 11(a). That is, the web 2 is a rectangle disposed on the upper side of the vertical part 4b of the T-shape of the fiber accumulated layer 4 over the area from and including the front portion to and including the rear portion with its width equal to that of the vertical part 4b.

Using the absorbent member according to the embodiments shown in Figs. 11(a) and 11(b) makes an absorbent article more compact and provides a better fit to the body of a wearer.

The absorbent member 1 of the embodiment shown in Fig. 12 has a fiber accumulated layer 4 shaped to letter "T" but with the stem of the T-shape split into two vertical parts 4b equal in width. The two vertical parts 4b are spaced at a distance equal to their width. The horizontal part 4a of the T-shaped fiber accumulated layer 4 is located on the rear portion of an absorbent article. The vertical parts 4b of the T-shape are disposed from and including the rear portion to and including the crotch portion of the absorbent article. There are no vertical parts 4b in the front portion. The web 2 is disposed in a rectangular configuration on the upper side of the vertical parts 4b of the T-shaped fiber accumulated layer 4 over the area from and including the front portion to and including the rear portion. The width of the web 2 is equal to the distance between the opposing, laterally outward edges of the vertical parts 4b. By the use of the absorbent member of the present embodiment, an absorbent article is easily designed to have both urine and fecal absorbency.

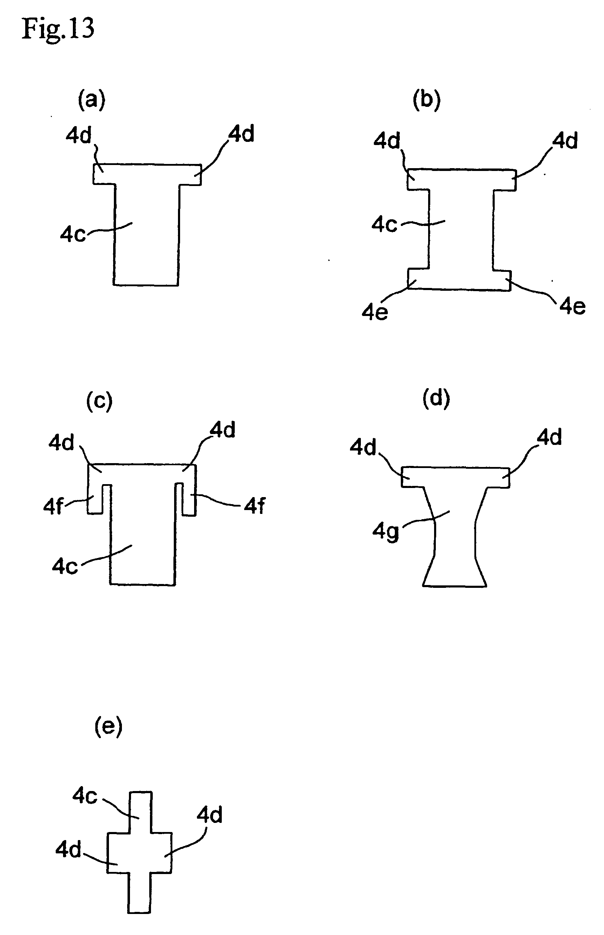

Figs. 13(a) through 13(d) represent other shapes of the fiber accumulated layer. The upper part of each shape illustrated in Figs. 13(a) to 13(d) is located in the front portion of an absorbent article, and the lower part in the rear portion. The fiber accumulated layer shown in Fig. 13(a) has a shape composed of a trunk 4c and a pair of rectangular ledges 4d extending laterally outward from the sides of the upper end portion of the trunk 4c. The fiber accumulated layer shown in Fig. 13(b) has a shape composed of a trunk 4c, a pair of ledges 4d, and a pair of rectangular ledges 4e extending laterally outward from the sides of the lower end portion of the trunk 4c. The fiber accumulated layer shown in Fig. 13(c) has a shape composed of a trunk 4c, a pair of ledges 4d, and a pair of rectangular pendants 4f hanging from the side portion of the ledges 4d. The fiber accumulated layer shown in Fig. 13(d) has a shape composed of an hourglass-shaped trunk 4g (with its lengthwise middle portion curved inward) and a pair of rectangular ledges 4d extending laterally outward from the sides of the upper end portion of the trunk 4g. The fiber accumulated layer shown in Fig. 13 (e) has a shape composed of a trunk 4c and a pair of rectangular ledges 4d extending laterally outward from the lengthwise middle part of the trunk 4c. In this way, a fiber accumulated layer can take on various shapes composed of a rectangular trunk extending in the longitudinal direction of an absorbent article and a pair of ledges extending laterally outward from the sides of the trunk.

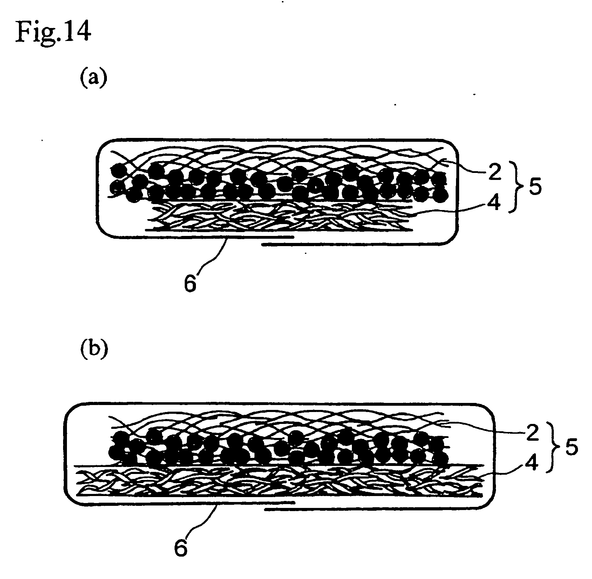

While, in the embodiment of Fig. 1, the web 2 containing the superabsorbent polymer 3 and the fiber accumulated layer 4 have the same width, they may have different widths. For example, the web 2 containing the superabsorbent polymer 3 may hang over both lateral side edges of the fiber accumulated layer 4 as illustrated in Fig. 14(a). Conversely, the fiber accumulated layer 4 may extend laterally outward from both lateral side edges of the web 2 containing the superabsorbent polymer 3 as illustrated in Fig. 14(b). The absorbent member of the embodiment shown in Fig. 14(a) is preferably used in disposable diapers for babies. Since the web 2 containing the superabsorbent polymer 3 is soft, widening the web 2 causes little discomfort in the crotch area. Improvement in absorbency can thus be achieved without discomfort or inadequate body fit. The absorbent member of the embodiment shown in Fig. 14(b) is desirably applied to a region having its area increased to secure absorption, such as the stomach region or buttocks. In particular, when it is disposed with a broad width in a region providing a fastening tape landing zone of a flat type disposable diaper having fastening tapes, the landing zone will have uniform thickness and stiffness over the whole area thereof. This is of importance to improve ease of fastening.

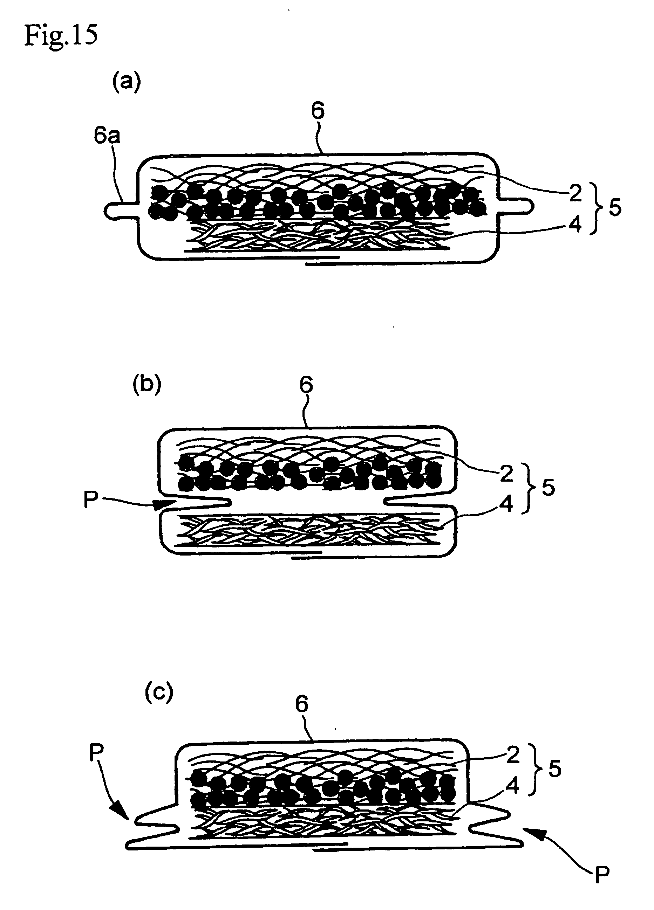

While, in the embodiment of Fig. 1, the fiber sheet 6 is wrapped around the absorbent core 5 to have the same width as the core 5, it may be loosely wrapped to form a bulge sticking out from both side edges of the absorbent core 5 as illustrated in Fig. 15(a). In this embodiment, the excess of the fiber sheet 6 forms a bulge 6a, with or without an adhesive in it, outboard of the lateral side edges of the absorbent core 5. By loosely wrapping the fiber sheet 6 around the absorbent core 5 so that the excess of the sheet 6 may form the bulge 6a on both sides of the core 5, the fiber sheet 6 has an allowance for swell of the absorbent core 5 on liquid absorption. This is effective to prevent the fiber sheet 6 from becoming a hindrance to the absorbent core's swelling when the absorbent core 5 absorbs a large quantity of urine.

The absorbent members shown in Figs. 15(b) and 15(c) produce the same effect as in the embodiment of Fig. 15(a). In Figs. 15(b) and 15(c), the fiber sheet 6 has pleats. The absorbent member of Fig. 15(b) has the fiber sheet 6 folded in the inside of the absorbent core 5 between the web 2 and the fiber accumulated layer 4 to form pleats P. The absorbent member of Fig. 15(c) has pleats P laterally sticking out from both side edges of the absorbent core 5.

In the embodiments illustrated in Figs. 15(a) to 15(c), the number and size (amplitude) of the bulges 6a and the pleats P are not limited and can be chosen as appropriate to the amounts of the superabsorbent polymer, web and fibers and the elongation of the fiber sheet.

The process of producing the absorbent members of the embodiments shown in Figs. 15(b) and 15(c) includes the step of folding the fiber sheet 6 in a zig-zag fashion (Z pattern). In the production of the absorbent member of the embodiment shown in Fig. 15(b), a web 2 is put on a fiber sheet 6, and a superabsorbent polymer 3 is sprinkled on the web 2. The fiber sheet 6 is folded inward to wrap part of the web 2 along the lateral sides of the web 2 and then folded outward in a Z pattern. A fiber accumulated layer 4 is stacked on the web 2, and the stack is completely wrapped in the fiber sheet 6.

In the production of the absorbent member of the embodiment shown in Fig. 15(c), a web 2 is put on a fiber sheet 6, and a superabsorbent polymer 3 is sprinkled on the web 2. A fiber accumulated layer 4 is superposed on the web 2 having the superabsorbent polymer sprinkled thereon. The fiber sheet 6 is folded in a Z pattern near the lateral side edges of the stack of the web 2 and the fiber accumulated layer 4 and then wrapped around the side edges of the stack to completely enclose the stack.

Other modifications of the absorbent member according to the present invention include a structure in which a plurality of laminates of the continuous fiber web 2 and the fiber accumulated layer 4 are stacked. The fiber accumulated layer can take on various shapes. For example, in the embodiments shown in Figs. 1 through 6, the fiber accumulated layer 4 may have a part thereof separated or may have a dart-shaped cutout.

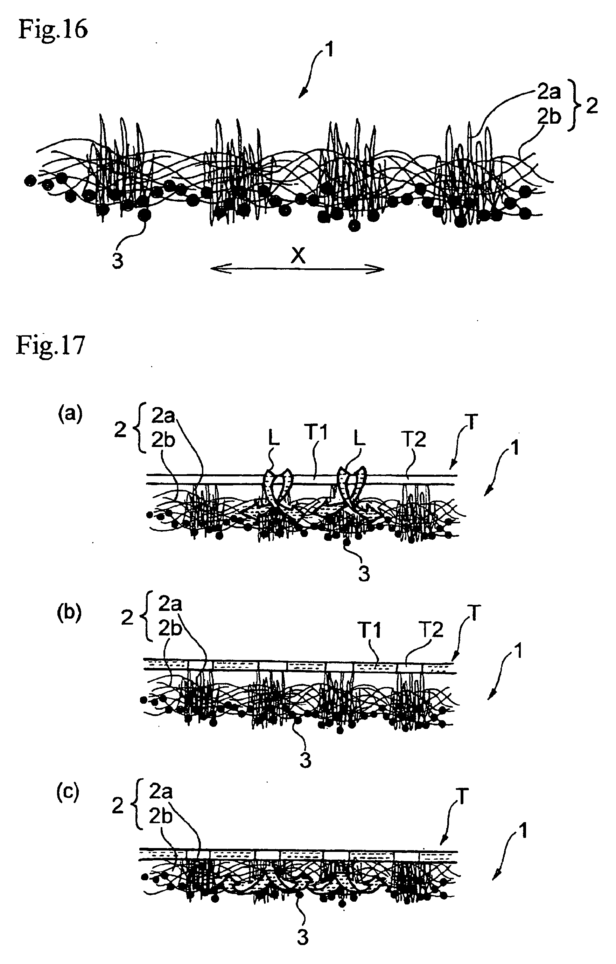

In Fig. 16 is illustrated yet another embodiment of the present invention. While not shown, the absorbent member 1 according to the present embodiment has a web 2 containing a superabsorbent polymer 3 covered with a fiber sheet. A single fiber sheet is used in the present embodiment. The fiber sheet covers the upper side of the web 2, wraps around the lateral sides of the web 2, and meets and overlaps with itself at the widthwise middle of the lower side of the web 2. Thus, the lower side of the web 2 is also covered with the fiber sheet.

The absorbent member 1 illustrated in Fig. 16 has continuous fibers oriented unidirectionally. The absorbent member 1 is characterized in that the web 2 has regions with a higher fiber content and regions with a lower fiber content, both regions extending in the orientation direction of the continuous fibers, and that these regions alternate in the direction perpendicular to the orientation direction of the continuous fibers.

The absorbent member 1 illustrated in Fig. 16 has continuous fibers oriented and extending in the direction perpendicular to the paper surface. The continuous fibers exist over the whole length of the absorbent member 1. Nevertheless, the continuous fibers do not have to exist over the whole length of the absorbent member 1. It is only necessary for the continuous fibers to exist in the target zone of the absorbent member 1 that is to face a point of body fluid discharge, provided that the continuous fibers satisfy the aforementioned requirement for fiber length.

The web 2 has regions 2a with a higher fiber content (hereinafter referred to as higher fiber content regions 2a) and regions 2b with a lower fiber content (hereinafter referred to as lower fiber content regions 2b), each extending in the orientation direction of the continuous fibers. The regions 2a and 2b alternate in parallel with each other and in the direction perpendicular to the continuous fiber orientation direction (direction X in Fig. 16). The higher fiber content regions 2a are regions having a relatively higher amount of fibers per unit area in a cross-section along the thickness direction of the web. The lower fiber content regions 2b are regions with a relatively lower amount of fibers per unit area of the web.

The width of each of the higher fiber content regions 2a and the lower fiber content regions 2b (the width in direction X in Fig. 16) is not critical in the present embodiment. The regions 2a and the regions 2b may have the same or different widths. When different, the width of the higher fiber content regions 2a may be larger than that of the lower fiber content regions 2a, or vice versa. In extreme cases, depending on the process of producing the web 2, the width of the lower fiber content regions 2b is much smaller than that of the higher fiber content regions 2a.

When seen through the topsheet, the absorbent member 1 of the present embodiment having the web 2 in which the higher and lower fiber content regions alternate in parallel with each other takes on a striped appearance attributed to the difference in amount of the fibers between these regions irrespective of their widths. The striped appearance gives a user a visual sensation of dryness. This tendency is stronger when the regions 2a and 2b extend in the longitudinal direction of the absorbent article.

While depending on the process of production of the web 2, it is preferred for the higher and lower fiber content regions 2a and 2b to have a width of 0.5 to 20 mm, more preferably 2 to 10 mm. The recited widths are preferred for securing clear visibility of the stripe pattern and for improving absorption performance. Improvement on absorption performance by the stripe design will be described later.

The change in fiber content between the higher and lower fiber content regions 2a and 2b may be either stepwise or gradual. The higher fiber content regions 2a preferably have a fiber content of 10 to 200 $g/m^2$, more preferably 20 to 100 $g/m^2$, while the lower fiber content regions 2b preferably have a fiber content of 20 $g/m^2$ or less, more preferably 3 to 10 $g/m^2$. The fiber content W1 in the higher fiber content region 2a is greater than the fiber content W2 in the lower fiber content region 2b. The fiber content W1 in the higher fiber content region 2a is preferably twice or more the fiber content W2 in the lower fiber content region 2b.

The fiber content is measured as follows. Prior to the measurement, a load of 24.5 kPa is applied to an absorbent article for 12 hours to make the absorbent article free from such influences as thickness recovery and wrinkles. A 10 cm by 10 cm specimen is cut out of the absorbent article and weighed to obtain the weight per unit area of the absorbent member. The amount of the superabsorbent polymer is determined, and the weight per unit area of the superabsorbent polymer is subtracted from the weight per unit area of the absorbent member to obtain an average weight per unit of the fibers of the absorbent member. The amount of the superabsorbent polymer in the absorbent member is determined by measuring the overall weight and area of the absorbent member, immersing the absorbent member in an ascorbic acid solution, followed by exposure to sunlight, to dissolve the superabsorbent polymer, washing away the dissolved polymer with water, and weighing residual fibers. The absorbent member is cut along a direction perpendicular to the continuous fiber orientation direction, and the higher fiber content regions and the lower fiber content regions are separated according to the fiber area ratio in the cut area. The absorbent member is cut with a cutter. A cut area corresponding to a width of 50 mm is dividedly scanned and inputted as image data. Each picture, taken of an area of 5 mm by 5 mm, is enlarged 25 times and inputted in sequence. The images are processed with Image Pro Plus software (Media Cybernetics) to calculate the area occupied by the fibers (the sum of the cross-sectional areas of the individual fibers) per image. The measurement was taken on five points to obtain an average. Assuming that the web has a uniform thickness, the area occupied by the fibers (referred to as an average area) is previously calculated from previously determined average weight per unit area of the fibers and the cross-sectional area of a single fiber. A region in which the fibers occupy an area equal to or more than the average area is regarded as a higher fiber content region, and a region in which the fibers occupy an area less than the average area is regarded as a lower fiber content region.

The numbers of the higher and lower fiber content regions 2a and 2b are not particularly limited. To ensure clear visibility of the stripe pattern and to improve absorption performance (hereinafter described), it is preferred that 3 to 50, more preferably 5 to 30, pairs of a higher fiber content region 2a and a lower fiber content region 2b be arrayed in parallel with each other in direction X in Fig. 16.

Since the web 2 has the higher and lower fiber content regions 2a and 2b, the "weight (per unit area) of the web 2" is an average weight of the whole web including both regions. The weight of the web 2 is preferably 120 to 400 $g/m^2$, more preferably 150 to 300 $g/m^2$, for applications to disposable diapers for babies; 35 to 200 $g/m^2$, more preferably 50 to 150 $g/m^2$, for applications to sanitary napkins; and 35 to 500 $g/m^2$, more preferably 50 to 400 $g/m^2$, for applications to incontinence pads.

As depicted in Fig. 16, the superabsorbent polymer is distributed almost uniformly in the direction perpendicular to the continuous fiber orientation direction, i.e., direction X in Fig. 16. Therefore, the ratio of the superabsorbent polymer to the fibers is higher in the lower fiber content region 2b than in the higher fiber content region 2a. To satisfy such a relation between the superabsorbent polymer and the fibers is important for achieving efficient liquid absorption, which will be described hereunder.

In Figs. 17(a) through 17(c) is shown liquid absorption mechanism by the absorbent member 1 of the present embodiment. A topsheet T is disposed on the absorbent member 1. The web 2 constituting the absorbent member 1 has higher fiber content regions 2a and lower fiber content regions 2b. The higher fiber content regions 2a come into contact with the topsheet T preferentially over the lower fiber content regions 2b because of their relatively high fiber contents. Accordingly, as shown in Fig. 17(a), the topsheet T will show different behaviors in drawing liquid between regions in contact and out of contact with the higher fiber content regions 2a (hereinafter referred to as web contact regions T2 and web non-contact regions T1, respectively). More specifically, when liquid L is discharged onto the topsheet T having the web non-contact regions T1, where no pressing force is imposed, and the web contact regions T2, liquid L is absorbed into the inside of the absorbent member 1 passing through the higher fiber content regions 2a in contact with the topsheet T (or the web contact regions T2) as illustrated in Fig. 17(b). Thereafter, the liquid then travels in the topsheet T from the web non-contact regions T1 to the web contact regions T2 by liquid's moving forces such as a diffusion force and a penetration force and then migrates into the inside of the absorbent member 1 through the higher fiber content regions 2a.

With a pressing force applied to the topsheet T, both the web contact regions T2 and the web non-contact regions T1 come in contact with the web 2 as illustrated in Fig. 17(c). When liquid L is discharged on the topsheet T in this state, a gradient of liquid drawing force is produced in the absorbent member because the higher fiber content regions 2a are compressed more than the lower fiber content regions 2b.

The above-mentioned liquid's moving forces and the gradient of liquid drawing force serve as driving forces to accelerate liquid migration from the web non-contact regions T1 through the web contact regions T2 to the higher fiber content regions 2a as illustrated in Figs. 17(b) and (17c) and also to make the liquid migrate from the web non-contact regions T1 to the lower fiber content regions 2b. As a result, the liquid hardly remains in the topsheet 2, and the topsheet 2 is easily kept in a dry state. Moreover, the stripe pattern made by the alternating higher and lower fiber content regions 2a and 2b adds a visual sensation of dryness.

In any of the states shown in Figs. 17(a) to 17(c), the liquid entering the absorbent member 1 is first absorbed by the superabsorbent polymer 3 present in the higher fiber content regions 2a. Because the higher fiber content regions 2a contain an increased amount of fibers relative to the amount of the superabsorbent polymer 3, the continuous fibers in the higher fiber content regions 2a enter between the particles of the superabsorbent polymer 3 which are swelling with absorption of the liquid. Gel blocking of the superabsorbent polymer 3 is thus suppressed. Furthermore, the continuous fibers function per se as a liquid guide to sustain absorbency. With body pressure application to the absorbent member 1 caused by, e.g., the wearer changing his/her posture as shown in Fig. 17(c), the liquid retained in the higher fiber content regions 2a is pressed to the lower fiber content regions 2b and absorbed by the superabsorbent polymer 3 present in the regions 2b. Since the continuous fibers are orientated in one direction, the liquid is also distributed along that direction.

In that way, the absorbent member 1 according to the present embodiment makes discharged liquid be absorbed in not only the spot where it is taken up but a wide area surrounding the spot. As a result, the entire area of the absorbent member 1 can be made use of to absorb body fluids, namely, efficient liquid absorption can be accomplished.

It is preferred for the superabsorbent polymer used in the present embodiment to have a liquid transit time of 20 seconds or less, more preferably 2 to 15 seconds, even more preferably 4 to 10 seconds, measured by the following method. The liquid transit time is measured as follows. A cylinder having a cross-sectional area of 4.91 cm$^2$ (inner diameter: 25 mm) with its bottom closable with a cock (inner diameter: 4 mm) is prepared. In the cylinder with its bottom closed is put 0.5 g of a superabsorbent polymer, and the cylinder was filled with physiological saline. After the polymer is swollen to saturation and sinks to the bottom, the cock is opened to have 50 ml of the physiological saline pass through. The time required for 50 ml of the saline to pass through is taken as the liquid transit time.

It is more preferred for the superabsorbent polymer used in the present embodiment to have high liquid permeability under load. More specifically, to effectively prevent gel blocking of the superabsorbent polymer from occurring, it is preferred for the superabsorbent polymer to have a liquid permeation rate of 30 to 300 ml/min, more preferably 32 to 200 ml/min, even more preferably 35 to 100 ml/min, as measured by the method described below. If the liquid permeation rate is less than 30 ml/min, the superabsorbent polymer particles swollen with liquid to saturation are liable to stick to one another under load and obstruct passage of liquid (gel blocking). The higher the liquid permeation rate, the more preferred to prevent gel blocking from occurring. Occurrence of gel blocking is prevented almost certainly where the liquid permeation rate is as high as about 300 ml/min. Where the liquid permeation rate exceeds 300 ml/min, the flow of the liquid in the absorbent member is too fast. It may follow that immobilization of the liquid is insufficient, which can cause leakage, particularly when a large amount of excrement is discharged at a time or when excrement is released very fast as by older babies or by adults, or when the absorbent member is designed to have a reduced thickness. In general, to increase the liquid permeation rate means to increase the degree of crosslinking of the superabsorbent polymer, which results in decreasing absorptive capacity of the superabsorbent polymer per unit weight. This leads to necessity to use an increased amount of the superabsorbent polymer. From these considerations, the upper limit of the liquid permeation rate shall be decided.

A superabsorbent polymer having a liquid permeation rate within the recited range can be obtained by, for example, controlling the crosslinking density in the inside and on the surface of the polymer particles, providing a crosslinking density gradient, or controlling the shape of the polymer particles.

The liquid permeation rate of a superabsorbent polymer is measured under a load of 2.0 kPa, which practically corresponds to the body pressure imposed to an absorbent member while an absorbent article is worn. A concrete method of measuring a liquid permeation rate is described, e.g., in JP-A-2003-235889, para. [0000]. In the present invention, the liquid permeation rate measurement is carried out by changing the sample weight from 0.200 g as specified in the above publication to 0.32 g. More specifically, the liquid permeation rate is measured according to the following procedures.

Method of measuring liquid permeation rate:

A filtration cylinder (inner diameter: 25.4 mm) equipped with a metal mesh (mesh size: 150 $\mu$m) and a narrow tube (inner diameter: 4 mm; length: 8 cm) with cock (inner diameter: 2 mm) is prepared. The cylinder with the tube closed with the cock is vertically held, and 0.32 g of a sample having a particle size adjusted to 150 to 850 $\mu$m is put therein.

Then, 50 ml of 0.9 wt% physiological saline is poured in the cylinder. After allowing the cylinder to stand for 30 minutes from the start of pouring the physiological saline, a circular rod weighing 21.2 g and having attached to one end thereof a metal mesh having a mesh size of 150 μm and a diameter of 25 mm is inserted in the filtration cylinder until the metal mesh comes into contact with the sample. One minute later, a 77.0 g weight is attached to the circular rod to apply an appointed load to the sample. After the cylinder is left to stand for an additional 1 minute period, the cock is opened, and the time T1 (sec) required for the liquid level of the saline to drop from the 40 ml mark to the 20 ml mark is measured. The liquid transit time is calculated according to equation below using the thus measured time T1 (sec). In the equation T0 is the time measured with no sample in the filtration cylinder.

$$\text{Liquid permeation rate (ml/min)} = 20 \times 60/(T1 - T0)$$

For more detailed account of the method of liquid permeation rate measurement, refer to JP-A-2003-235889, paras. [0008] and [0009]. The measuring equipment is illustrated in Figs. 1 and 2, ibid.

The liquid transit time is a measure of the gel strength of the superabsorbent polymer. The shorter the liquid transit time, the higher the gel strength. The superabsorbent polymer that can be used is not particularly limited as long as the above-mentioned characteristics are satisfied. Exemplary superabsorbent polymers include sodium polyacrylate, acrylic acid-vinyl alcohol copolymers, crosslinked sodium polyacrylate, starch-acrylic acid graft copolymers, isobutylene-maleic anhydride copolymers and saponification products thereof, potassium polyacrylate, and cesium polyacrylate. In order for the superabsorbent polymer to satisfy the characteristics, a crosslinking density gradient is provided on the surface of the polymer particles, or aspherical, irregularly shaped superabsorbent polymer particles are used. Specifically, the methods disclosed in U.S. Patent 5,865,822, col. 10, 1. 62 to col. 12, 1. 40 can be used.

The web 2 may contain other organic or inorganic particles than the superabsorbent polymer 3, including activated carbon, silica, alumina, titanium oxide, and various clay minerals (e.g., zeolite, sepiolite, bentonite, and cancrinite), as deodorants or antimicrobials. The inorganic particles may have part of the metal sites displaced. Various organic or inorganic buffers can also be used, including acetic acid, phosphoric acid, citric acid, succinic acid, adipic acid, malic acid, lactic acid, and salts of these acids, either individually or as a mixture thereof. Various amino acids are also used. The function of these ingredients is to suppress the smell of the fluids absorbed by the absorbent member and the smell of the constituent materials per se. The organic or inorganic buffers also have a function of neutralizing excrement, for example ammonia generated by decomposition of urine to maintain the diaper neutral to weakly acidic, which minimizes the influences on the skin even if rewet occurs. Furthermore, the organic or inorganic buffers are expected to protect such continuous fibers as have an intermolecular ester bond (e.g., cellulose acetate fibers) against damage due to ester bond cleavage by an alkali.

The web 2 may further contain hydrophilic fine powder or stable fiber to improve the liquid retention, rate of absorption, and dryness. The hydrophilic fine powder or staple fiber includes fibrillated or non-fibrillated cellulose powder, carboxymethyl cellulose and metal salts thereof, carboxyethyl cellulose and metal salts thereof, hydroxyethyl cellulose and derivatives thereof, silk powder, nylon powder, and rayon, cotton or wool staple fibers. Preferred of them is cellulose powder for being the most effective of the others. The hydrophilic fine powder or staple fiber may be dispersed on the web 2 before the superabsorbent polymer 3 is sprinkled or be mixed with the superabsorbent polymer 3 to be sprinkled on the web 2.

The absorbent member 1 having the above-described structure is thin, light weight, and highly breathable. The degree of breathability of the absorbent member 1 is expressed in terms of air permeation resistance per 100 g superabsorbent polymer per square meter. The air permeation resistance of the absorbent member 1 is as small as 0.4 kPa.s/m or less. A smaller the air permeation resistance means higher breathability. The air permeation resistance given above is such an extremely small value as about 1/2 of that of a conventional absorbent member composed of fluff pulp and a superabsorbent polymer. There is no particular lower limit to the air permeation resistance. The lower the value, the higher the breathability. Air permeation resistance is measured with an air permeability tester KES-F8 from Kato Tech Co., Ltd. KES-F8 sends air at a constant flow rate (4 (cc·cm$^2$)/sec) to a sample to measure the pressure loss.

Some of the absorbent members according to the present invention have an air permeation resistance lower than the detection limit of the above-described tester, i.e., about 0.2 kPa·s/m. Included in such absorbent members are those containing no pulp as a constituent material but having a superabsorbent polymer-containing continuous fiber web wrapped in tissue paper. Accordingly, there is no lower limit to the air permeation limit in the present invention. Examples of other materials providing an air permeation resistance lower than the detection limit of the tester include ordinary tissue paper used in absorbent articles, topsheets, and gauze.

The absorbent member shown in Fig. 16 preferably has a compression work (WC) of 0.98 cN.cm/cm$^2$ or more, more preferably 1.47 cN.cm/cm$^2$ or more, as a whole as measured with a KES compression tester in the thickness direction. The compression work is measured with a handy-type compression tester KES-G5 from Kato Tech Co., Ltd.

as follows. A specimen measuring 5 cm by 10 cm is attached to the mount of the tester. The specimen is compressed with a steel plate having a 2 cm$^2$ circular plane at a compression speed of 20 $\mu$m/sec. The maximum compressive load is set at 4.9 kPa. The measurement in the recovery process is carried out at the same speed. The compression work WC is represented by formula:

$$WC = \int_{T0}^{Tm} PdT$$

When the absorbent member 1 shown in Fig. 16 has a compression work WC in the above recited range, one advantage offered is that an absorbent article using the absorbent member causes no discomfort and exhibits excellent recovery on release from pressure (e.g., on removal from the package) to restore cushioning properties. Another advantage is that the absorbent article fits the body of a wearer even immediately after taken out of the package and shows properties of rapidly absorbing fluids. Still another advantage is that the absorbent member can be compressed to make the absorbent article very compact.

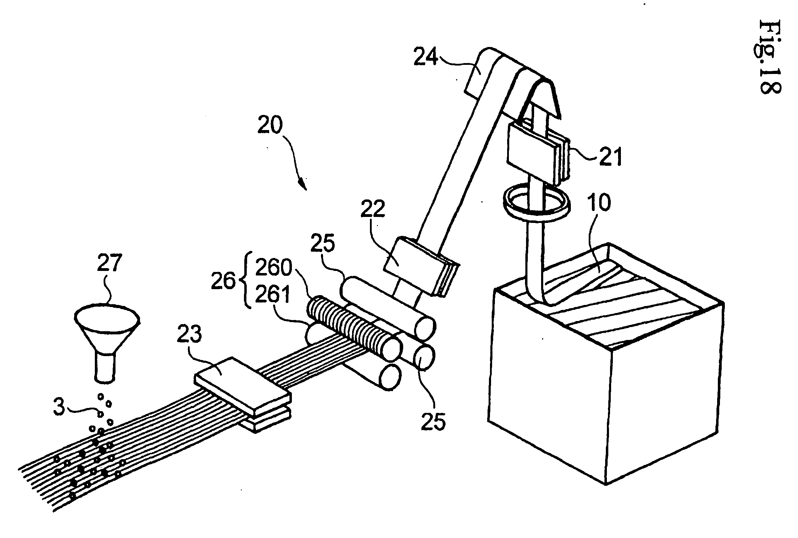

A process of making the absorbent member 1 shown in Fig. 16 will be described with reference to Fig. 18, in which apparatus used advantageously to produce the absorbent member 1 is illustrated. The apparatus has an opening means 20 for successively opening continuous fibers 10 during conveyance.

The opening means 20 has banding jets 21, 22, and 23 to successively spread the continuous fibers 10 during conveyance. The opening means 20 also has a guide 24 between the banding jets 21 and 22 to once raise and then lower the stream of the continuous fibers 10. The opening means 20 further has a feed unit 25 and a blooming unit 26 between the banding jets 22 and 23.

Each of the banding jets 21, 22, and 23 is a unit for opening and laterally spreading the running stream of continuous fibers 10 by blowing air. The feed unit 25 includes a pair of rollers that nip the continuous fibers 10 opened by the banding jet 22 and feed them at a prescribed speed. The blooming unit 26 includes a roller 260 having a large number of discs arrayed around its periphery in an axial direction at a given interval and an anvil roller 261, between which the tensioned continuous fibers 10 are combed. A means 27 for feeding a polymer is provided downstream the banding jet 23. The polymer feeding means 27 is a unit for evenly sprinkling a prescribed amount of a superabsorbent polymer 3 on the bloomed continuous fibers from the blooming unit 26.

In the process of making the absorbent member 1 by the use of the apparatus having the above-described configuration, the continuous fibers 10 are fed under a prescribed tension. Having crimp as stated previously, the continuous fibers 10 easily stretch in the machine direction under tension. In this state, a tow of the continuous fibers 10 is opened laterally to increase its width by the banding jets 21, 22, and 23 of the opening means 20. Lateral spreading of the continuous fibers 10 can be achieved smoothly by effecting the spreading in divided stages with a plurality of banding jets.

The continuous fibers 10 are combed and formed into higher fiber content regions and lower fiber content regions by the blooming unit 26 located between the banding jets 22 and 23. The amounts of fibers in the higher and lower fiber content regions are decided primarily by the thickness and pitch of the discs around the roller 260. The widths of the higher and lower fiber content regions are decided primarily by the degree of opening the tow by the banding jet 23 provided downstream the blooming unit 26.

The superabsorbent polymer 3 is sprinkled over the continuous fibers 10 having the higher and lower fiber content regions. Before sprinkling, the tension of transporting the continuous fibers 10 is lessened to release the continuous fibers from the stretched state. On release from tension, the continuous fibers return to their crimped state. In this state, the superabsorbent polymer 3 is sprinkled. The crimped continuous fibers have interfiber spaces in which the superabsorbent polymer 3 can be retained. The superabsorbent polymer 3 is thus embeddedly supported in the spaces. In contrast, it is not easy to embed and retain the superabsorbent polymer 3 among the tensioned and stretched continuous fibers 10 due to a failure to form ample spaces enough to hold the superabsorbent polymer 3. It is effective in helping the polymer 3 be embeddedly retained to suck the continuous fibers 10 from the reverse side simultaneously with sprinkling the superabsorbent polymer 3. It is possible to vary the distribution of the polymer 3 in the web thickness direction by appropriately adjusting the degree of suction.

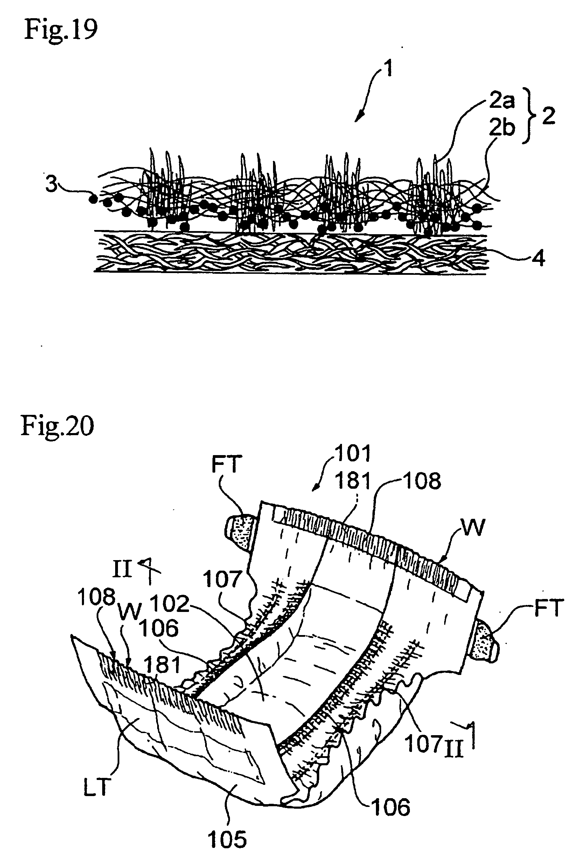

The absorbent member 1 of the embodiment illustrated in Fig. 19 is a modification of the absorbent member of Fig. 16. The absorbent member 1 illustrated in Fig. 19 has a fiber accumulated layer 4 superposed on the lower side of the web 2. The fiber accumulated layer 4 may or may not contain a superabsorbent polymer. The description on the fiber accumulated layer of the absorbent member shown in Fig. 1 appropriately applies to the fiber accumulated layer 4. In Fig. 19, the fiber sheet is not depicted.

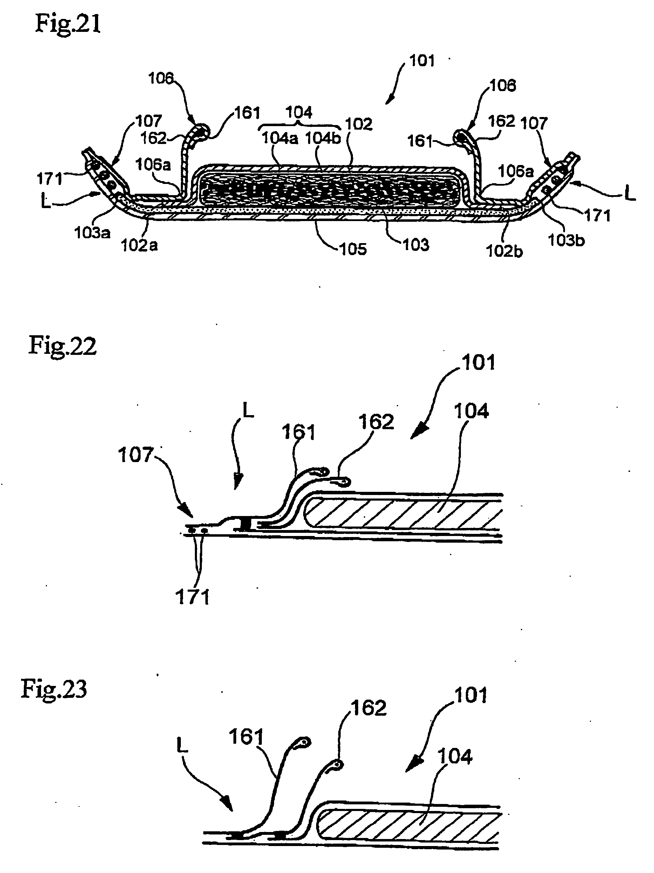

Fig. 20 shows an embodiment of the absorbent article according to the present invention. Fig. 20 represents application of the absorbent article of the present invention to a diaper. Fig. 21 is a cross-section of Fig. 20 taken along line II-II. The present embodiment is characterized in that the absorbent member has an air permeation resistance of 0.4 kPa·s/m or less per 100 g superabsorbent polymer per m$^2$. The diaper 101 of the present invention has a liquid permeable topsheet 102, a liquid impermeable or water repellent (hereinafter inclusively referred to as "liquid impermeable") backsheet 103, and a liquid retentive absorbent member 104 intermediate between these two layers. The diaper 101 is substantially a rectangle longer than wide.

The topsheet 102 that can be used includes nonwoven fabrics, perforated nonwoven fabrics, and perforated films. The topsheet 102 has breathability as well as liquid permeability. The backsheet 103 has moisture permeability as well as liquid impermeability. The terminology "breathability" as used herein denotes in principle properties relating to air permeability. The terminology "moisture permeability" as used herein means in principle properties relating to water vapor transmissivity or permeability. To have "breathability" means to have permeability to not only air but water vapor. Likewise, to have moisture permeability means to have permeability to not only water vapor but air.

The diaper 101 has its both lateral side edges curved inward in an arc in the crotch portion thereof, having as a whole the shape of a sandglass with the longitudinally middle portion thereof narrowed. Both the topsheet 102 and the backsheet 103 extent outward from both lateral side edges and both longitudinal ends of the absorbent member 104. The topsheet 102 is narrower than the backsheet 103 in the width direction. Accordingly, the lateral side edges 102a and 102b of the topsheet 102 are positioned inboard of the lateral side edges 103a and 103b of the backsheet 103.

A breathable exterior sheet 105 is disposed on the outer side of the backsheet 103. The exterior sheet 105 and the backsheet 103 are joined with, e.g., a hot melt adhesive. The hot melt adhesive is applied discontinuously, e.g., by bead coating or spiral coating, so as not to impair moisture permeability possessed by the backsheet 103. The exterior sheet 105 extends outward from both lateral side edges and both longitudinal ends of the topsheet 102 and the backsheet 103 and defines the outline of the diaper 101. The exterior sheet 105 is made of, for example, various nonwoven fabrics.

A pair of standing gathers 106 and a pair of leg gathers are formed on both lateral sides of the diaper 101 by disposing the respective elastic members. All the gathers 106 and 107 extend in the longitudinal direction of the diaper 101. The standing gathers 106 are each formed of a standing gather-forming sheet material 162 having an elastic member 161. The standing gathers 106 function as a barrier for preventing a fluid not absorbed by the absorbent member 104 and spreading laterally from spreading laterally any farther. Therefore, the sheet material 162 forming the standing gathers 106 is preferably water-repellent. It is also preferred for the sheet material 162 to have breathability to suppress a rise in humidity in the diaper 101 while worn.

The diaper 101 may have two or more pairs of standing gathers for the following reason. To secure breathability of the absorbent member 104 and its vicinities, it is desirable for the absorbent member to have a reduced thickness and an average density decreased as much as possible. However, it is likely that such an absorbent member has little reserve capacity for temporarily accommodating a liquid when a large amount of urine is discharged or when urine is discharged at a high rate (for example, when the wearer is an older baby or an adult). Therefore, it is a preferred embodiment to provide two or more pairs of standing gathers for ensuring leakage prevention. The details of an absorbent article with two or more standing gathers will be described later.

From the above-mentioned various standpoints, the sheet material 162 is preferably selected from a nonwoven fabric, a perforated film, etc. that are essentially water repellent or have been rendered water repellent. Nonwoven fabrics are particularly preferred. Examples of preferred nonwoven fabrics include thermal-bonded nonwoven fabrics, spun-bonded nonwoven fabrics, spunbonded-meltblown-spunbonded nonwoven fabrics, spunbonded-meltblown-meltblown-spunbonded nonwoven fabrics made of single-component fibers of thermoplastic resins, such as polyethylene, polypropylene, and polyethylene terephthalate, or conjugate fibers thereof.

The standing gather-forming sheet material 162 may be a composite composed of the nonwoven fabric and an elastic substance. The sheet material 162 may also be a sheet obtained by incorporating a fibrous elastomeric material into the nonwoven fabric and then elasticating the nonwoven fabric stretchy. The elastication can be achieved by, for example, combining the elastomer and the nonwoven fabric with no stretch and slitting the nonwoven fabric or applying a kind of breaking treatment to the nonwoven fabric.

The sheet material 162 is longer than is wide, extending in the longitudinal direction of the diaper 101. The sheet material 162 has an elastic member 161 fixed in its stretched stated along one lateral side edge thereof. The sheet material 162 is joined to the topsheet 102 along the longitudinal direction of the diaper 101 at a position outboard of each lateral side edge of the absorbent member 104. The joint of the sheet material 162 and the topsheet 102 is the base of the standing gather 106.

It is preferred for the sheet material 162 to extend laterally outward from the base 106a and be joined to a moisture permeable sheet in the outboard extension. For example, the sheet material 162 is preferably joined directly to the backsheet 103 as illustrated in Fig. 21 to ensure prevention of liquid diffusion by the standing gathers 106 and to improve leakage resistance of the diaper 101. More specifically, the sheet material 162 extends laterally outward from the base 106a and joined directly to the backsheet 103 within the extension. The sheet material 162 is also joined to the exterior sheet 105 along the outboard edge of its extension. There are thus provided leg flaps L (see Fig. 21). Each leg flap L has one or, in this particular embodiment, more than one leg elastic members 171 disposed therein almost linearly to make a leg gather 107. The leg elastic members 171 are held between the sheet material 162 and the exterior sheet 105 in a region defined by the joint between the extension of the sheet material 162 and the backsheet 103 and the joint between the extension and the exterior sheet 105.

As previously described, the sheet material 162 has breathability, the exterior sheet 105 has breathability, and the backsheet 103 has moisture permeability. Accordingly, the leg flaps L possess breathability or moisture permeability. In other words, the diaper 101 is breathable and moisture permeable in its regions laterally outside of the absorbent member 104, specifically in its regions between each side edge of the absorbent member 104 and the corresponding outermost leg elastic member 171.

The configuration of both lateral side portions of the diaper 101 is as described above. The following is the configuration of both longitudinal end portions of the diaper 101. The topsheet 102, the backsheet 103, and the exterior sheet 105 extend longitudinally outward from the longitudinal ends of the absorbent member 104 and joined all together in their extensions to form opposing waist flaps W (see Fig. 20). All the sheets forming the waist flaps W have breathability or moisture permeability, and the waist flaps W are breathable or moisture permeable accordingly.

The waist flap W has one or more than one waist elastic members 181 (one elastic member 181 in the present embodiment) disposed thereon along the diaper's width direction to form a waist gather 108. As stated, since the waist flap W is breathable or moisture permeable, the region between the outermost waist elastic member 181 and the corresponding edge of the absorbent member 104 is breathable or moisture permeable. While in the present embodiment a waist elastic member is disposed on both the opposing waist flaps W, the diaper 101 may have the waist elastic member on only one of the waist flaps W depending on the use of the diaper.

The diaper 101 has a pair of fastening tapes FT attached to the lateral sides of one of the longitudinal end portions thereof and a landing tape LT attached to the exterior sheet 105 in the opposite end portion thereof. The fastening tapes FT and the landing tape LT are designed so that the former is fastened to the latter while the diaper 101 is worn. For example, a hook and loop fastener can be used as the fastening tapes FT and the landing tape LT.

As illustrated in Fig. 21, the absorbent member 104 of the diaper 101 has a web 104a of hydrophilic continuous fibers. The web 104a has a superabsorbent polymer 104b embeddedly supported therein.

The absorbent member 104 having the above-described structure is thin, light weight, and highly breathable. The breathability of the absorbent member 104 is preferably, being expressed in terms of air permeation resistance per 100 g superabsorbent polymer per m$^2$, 0.4 kPa·s/m or less, more preferably 0.3 kPa·s/m or less. A smaller air permeation resistance means higher breathability. The air permeation resistance given above is such an extremely small value as about 1/2 of that of a conventional absorbent member having a fiber accumulated member of fluff pulp and a superabsorbent polymer. There is no particular lower limit to the air permeation resistance. The lower the value, the higher the breathability. Air permeation resistance is measured with an air permeability tester KES-F8 (trade name) from Kato Tech Co., Ltd. KES-F8 sends air at a constant flow rate (4 (cc·cm$^2$)/sec) to a sample to measure the pressure loss. The air permeation resistance value as measured is reduced to a value per 100 g of the superabsorbent polymer per square meter.

Some of the absorbent members according to the present invention have an air permeation resistance lower than the detection limit of the above-described tester, i.e., about 0.2 kPa·s/m. Included in such absorbent members are those containing no pulp as a constituent material but having a superabsorbent polymer-containing continuous fiber web wrapped in tissue paper. Accordingly, there is no lower limit to the air permeation limit in the present invention. Examples of other materials providing an air permeation resistance lower than the detection limit of the tester include ordinary tissue paper used in absorbent articles, topsheets, and gauze.

The absorbent member 104 containing the continuous fiber web 104a exhibits high breathability in both the thickness direction and the planar direction. Therefore, while the diaper 101 is worn, water vapor released from the wearer's body effectively escapes from the diaper 101 through the absorbent member 104. As a result, an increase in humidity inside the diaper is effectively prevented.

There are roughly two pathways in the diaper 101 according to the present embodiment for water vapor to escape; one in the thickness direction and the other in the planar direction. The escape pathway in the thickness direction is one through which water vapor moves through the thickness of the absorbent member 104 and then passes through the backsheet 103 having water vapor transmissivity (moisture permeability) and the exterior sheet 105 having breathability, thereby getting out of the diaper 101. To help water vapor escape through this pathway more effectively, it is preferred that the backsheet 103 have a water vapor transmission rate (JIS Z0208) of 0.6 to 3.5 g/(100 cm$^2$·hr), more preferably 1 to 3 g/(100 cm$^2$·hr). With the moisture permeability falling within the recited range, the backsheet 103 secures sufficient water vapor permeability to prevent overhydration or skin rash from occurring while preventing the liquid absorbed by the absorbent member 104 from oozing out. The water vapor transmission rate is measured in accordance with JIS Z0208 (dish method) under conditions of 30°C/90% RH.

The sheet having water vapor permeability can be, for example, a porous sheet. A porous sheet is obtained by stretching a sheet molded from a molten resin composition. The porous sheet is preferably designed to have sufficient moisture permeability while maintaining moldability and sheet strength. The resulting porous sheet may be subjected to embossing or various surface treatments if desired. The porous sheet may be used as a composite sheet with other sheeting, such as an exterior sheet 105, e.g., a nonwoven fabric or paper.

The escape pathway in the planar direction is one through which water vapor moves in the absorbent member 104 in the planar direction of the absorbent member 104 and then passes from the sides and ends of the absorbent member 104 through the leg flaps L and the waist flaps W, thereby getting out of the diaper 101. As previously mentioned, since the leg flaps L and the waist flaps W of the diaper 101 according to the present embodiment have breathability or moisture permeability, water vapor is allowed to escape smoothly through these flaps. In the leg flaps L, in particular, since the lateral side edges of the topsheet 102 are inboard of the corresponding lateral side edges of the backsheet 103, the number of the sheet materials forming the leg flaps L is reduced by one. As a result, the leg flaps L have accordingly improved breathability or moisture permeability. To help water vapor escape through the leg flaps L and the waist flaps W more smoothly, where the sheets are joined with, e.g., a hot melt adhesive to form these flaps, the adhesive is preferably applied patternwise, such as by bead coating or spiral coating, so as to minimize reduction in breathability. A coating system forming a film of an adhesive covering the entire surface to be joined is unfavorable. When applied with a coater, a hot melt adhesive is preferably applied in a comb teeth pattern or intermittently. When applied to the entire surface, it is preferred to sufficiently reduce the amount of the hot melt adhesive per unit area to cause scratches or skips where the adhesive is not applied. In order to minimize reduction of breathability and yet to develop proper adhesive strength, the amount of the hot melt adhesive to be applied is preferably 3 to 30 $g/m^2$, more preferably 5 to 20 $g/m^2$.

The web 104a-containing absorbent member 104 having an air permeation resistance not more than the above recited value can be obtained by, for example, proper selection of the weight of the continuous fibers in the web 104a, the thickness of the web 104a, and the weight of the superabsorbent polymer. The inventors' researches have revealed that a proper range of the weight of the web 104a is from 5 to 200 $g/m^2$, desirably 10 to 100 $g/m^2$, and that a proper range of the weight of the superabsorbent polymer to be sprinkled is 50 to 500 $g/m^2$, desirably 100 to 300 $g/m^2$.

The total weight of the web 104a and the superabsorbent polymer 104b in the absorbent member 104 preferably ranges from 55 to 700 $g/m^2$, more preferably from 110 to 400 $g/m^2$.

The air permeation resistance of the absorbent member is also affected by the pattern and amount of application of an adhesive, e.g., a hot melt adhesive, used to join various members constituting the absorbent member. To let water vapor escape more smoothly, a hot melt adhesive is preferably applied patternwise by bead coating, spiral coating or spray coating so as to minimize reduction of breathability. In order to minimize reduction in breathability and yet to develop proper adhesive strength, the amount of the hot melt adhesive to be applies is preferably in a range of from 3 to 30 $g/m^2$, more preferably from 5 to 20 $g/m^2$.

Besides the conditions concerning the hot melt adhesive, factors influential on the air permeation resistance of the absorbent member include the thickness and density of the absorbent member. In order for a superabsorbent polymer to be supported in an opened mass of fluff pulp without resulting in an extremely hard absorbent member, it is necessary to use fluff pulp as much as the superabsorbent polymer. This means that an attempt to increase absorbency results in an increase in thickness of the absorbent member. The air permeation resistance of the absorbent member increases accordingly. If the amount of the fluff pulp is decreased to reduce the absorbent member's thickness, the absorbent member should be compressed to ensure polymer supporting capability. As a result, the absorbent member decreases in thickness but increases in density, resulting in an increased air permeation resistance.

Compared with the conventional absorbent member containing fluff pulp as a main component, the absorbent member 104 having the web 104a provides a less dense structure with larger interstices between fibers and therefore exhibits good liquid permeability. When the absorbent member 104 is slow to absorb a liquid, it is likely to happen that a liquid fails to be sufficiently absorbed by the absorbent member 104 because it passes through the absorbent member 104 before being absorbed by the superabsorbent polymer 104b. Taking this into consideration, it is desirable for the superabsorbent polymer 104b contained in the web 104a to have a sufficiently high absorption rate whereby the liquid is contained within the absorbent member 104 without fail. The absorption rate of the superabsorbent polymer 104b is generally represented in the art by the value obtained by the demand wettability (DW) method. An absorption rate (ml/(0.3 g·30 sec)) by the DW method can be measured with a DW tester generally known for carrying out the DW method. In some detail, with the liquid levels of physiological saline being equal, 0.3 g of a superabsorbent polymer is scattered on a mount (diameter: 70 mm; No. 1 glass filter having placed thereon No. 2 filter paper), and the water absorption after 30 seconds is gauged by reading the scale on the buret indicating a drop of the liquid level of physiological saline (the water absorption at the time of scattering the polymer is taken zero). The amount of absorption as measured is taken as an absorption rate.

The superabsorbent polymer 104b which is preferably used in the present embodiment has an absorption rate of 2 to 10 ml/(0.3 g.30 sec), more preferably 4 to 8 ml/(0.3 g.30 sec), measured by the DW method. In the production of conventional absorbent members made mainly of fluff pulp, the use of a superabsorbent polymer having such a high absorption rate has been avoided for fear of inducing gel blocking which can lead to leakage. In the present embodiment, since the web 104a has a less dense structure, the superabsorbent polymer having such a high rate of absorption hardly causes gel blocking and, on the contrary, effectively prevents leakage.

The web 104a preferably has a density of 0.005 to 0.20 g/cm$^3$, more preferably 0.01 to 0.10 g/cm$^3$, in relation to the absorption rate of the superabsorbent polymer. Having the density falling in that range offers the following advantages. The absorption rate in the web 104a can be controlled within a range appropriate to the absorption rate of the superabsorbent polymer. Softness of the absorbent member is secured. The fibers of the web have an appropriate interfiber distance to exhibit improved capabilities of supporting fine superabsorbent polymer particles.

Among means for preventing side leakage more effectively is to use straight continuous fibers; for such continuous fibers are superior to crimped continuous fibers in liquid distribution in the orientation direction of the continuous fibers. From this viewpoint, it is preferred that the web 104a be composed of a plurality of layers and that the continuous fibers in a part of at least one of the layers be held in their stretched and straightened state. Where the web 104a has a dual layer structure, the crimped continuous fibers located in a part, e.g., a longitudinally middle part, of the topsheet side layer can be held in a straightened state thereby to guide a liquid preferentially in the longitudinal direction of the absorbent member. The same effect can be obtained by using a web of non-crimped, hydrophilic continuous fibers in addition to the web of crimped continuous fibers. For instance, after a superabsorbent polymer is sprinkled on the web of crimped continuous fibers, a web of non-crimped continuous fibers can be disposed on the polymer-sprinkled side of the crimped fiber web.

It is desirable that the absorbent member 104 be flexible. When a value "handle" measured with a handle-o-meter is adopted as a measure of flexibility, the "handle" of the absorbent member 104 is preferably 4 N or less, more preferably 2 N or less. The measurement with a handle-o-meter is as follows. JIS L1096 (stiffness and softness measuring method) is followed. A 50 mm by 150 mm specimen is cut out of an absorbent member 104 with the length and width coinciding with those of the absorbent member. The specimen is placed on the platform having a 60 mm wide slot with the length perpendicular to the slot. The force required for a 2 mm thick penetrator blade to force the center of the specimen into the slot is read. In the present invention, a handle-o-meter HOM-3 available from Daiei Kagaku Seiki Co., Ltd. is used. The measurement is made on three points to obtain an average.

While not illustrated, the absorbent member 104 according to the present embodiment has the web 104a and the superabsorbent polymer 104b wrapped in a fiber sheet. The absorbent member 104 may have a fiber accumulated layer superposed on the upper side and/or the lower side of the web 104a containing the superabsorbent polymer 104b. The fiber accumulated layer and the web 104a having the polymer 104b may be wrapped as a unit in a fiber sheet. Whatever configuration the absorbent member 104 may take on, it is preferred for the absorbent member 104 to have an air permeation resistance falling within the above recited range. The absorbent member 104 is preferably of a thin type with a thickness of 1 to 4 mm, more preferably 1.5 to 3 mm, from the standpoint of securing sufficient breathability.

In another embodiment of the absorbent member, a second web of hydrophilic continuous fibers containing no superabsorbent polymer is superposed on the upper side and/or the lower side of the web 104a having a superabsorbent polymer embeddedly supported therein. Where the continuous fibers of the second web are crimped fibers, particularly where the crimped continuous fibers have the above-recited crimp percentage, the second web acts as a cushioning layer against pressing in the thickness direction to impart improved wearing comfort to the absorbent article. Moreover, the interfiber space in the absorbent member increases to improve breathability. Furthermore, because the absorbent member possesses excellent recovery from compression, a plurality of the absorbent articles, such as diapers, can be made thinner by compression packing in a package bag, and yet each article rapidly restores its original thickness after taken out of the package.

Recovery from compression of the absorbent member freed from the influences of the gathers is preferably such that the thickness after one hour from release from pressure is at least 1.2 times, more preferably 1.5 times or more, the initial thickness. The initial thickness of the absorbent member is the thickness measured after a load of 250 g/cm$^2$ is applied for 12 hours. The thickness of a stack of five samples is measured and divided by five to give a thickness per sample. Thickness measurement is taken with a load of 2.5 g/cm$^2$ applied to the stack by placing a 5 cm square acrylic resin plate and a weight. A laser displacement sensor LK080 class 2 available from Keyence Corp. is used. Measurement is conducted at five positions to obtain an average. If a piece of data fluctuates 20% or more, the piece is tossed out and replaced with an additional one.

Part of the absorbent member may be perforated to further improve the breathability. Being of thin type, the absorbent member of the present embodiment is easier to perforate than conventional thick type absorbent members. Otherwise, the absorbent member of the present embodiment may be composed of a plurality of segments with a groove between adjacent segments. In this configuration, the web may be continuous or discontinuous between the segments.

Modifications of the diaper according to the embodiment shown in Figs. 20 and 21 will be described by referring to Figs. 22 through 26. Unless particularly noted, the description of the embodiment shown in Figs. 20 and 21 applies to the embodiments shown in Figs. 22 to 26. The members common to Figs. 20, 21, and 22 to 26 are given the respective same numerical references.

The diaper 101 of the embodiment illustrated in Fig. 22 has two pairs of opposing standing gathers. The diaper 101 has a leg gather 107 formed of a leg flap L extending laterally outward from the side edge of the absorbent member 104 and leg elastic members 171 extending in the longitudinal direction of the diaper 101 and disposed in their stretched state along the edge of the leg flap L. The diaper 101 also has a first standing gather 161 and a second standing gather 162 each having its base between the leg gather 107 and the corresponding side edge of the absorbent member 104. The first standing gather 161 is closer to the leg gather, and the second standing gather 162 to the absorbent member.

The three gathers located in each leg flap L are preferably designed such that the outermost one may have a higher contractibility than the rest of three. That is, the contractive forces of the leg gather 107, the first standing gather 161, and the second standing gather 162 being taken as L1, L2, and L3, respectively, a preferred relationship is L1>L2 and L1>L3. It is more preferred that the contractive force gradually decreases from the outermost to the innermost, i.e., L1>L2>L3 for the following reasons.

Absorbent articles have been designed based on the concept that thickness reduction without causing leakage could be accomplished by providing gathers having high contractibility so as not to leave a gap between a wearer's body and the absorbent article. However, strongly contractible gathers tend to leave marks on the skin and, when combined with a thin and flexible absorbent member as provided by the present invention, tend to curl up the absorbent article, making it difficult to put the absorbent article on a wearer. Moreover, too strong contractive force of gathers creates downward force to cause displacement. These inconveniences associated with conventional absorbent articles can be averted by providing a pair of leg gathers and two or more pairs of standing gathers with their contractibility satisfying the above-described relationship.

The contractive force of a gather is measured as follows. A specimen cut out of a gathered part is tested on a tensilon tester ORIENTEC RTC-1150A to plot a hysteresis curve. The stress in the retracting curve is taken as a contractive force. The pulling and retracting speeds were 300 mm/min. The initial span length of the specimen is 100 mm, and the maximum elongation is 100 mm (stretched to double the initial length). The stress at 50 mm back from the maximum stretched length in the retracting curve of the hysteresis is read as a contractive force of the specimen. Measurement is made on five specimens per sample to obtain an average. When the maximum elongation does not reach 100 mm, the stress required for stretching the specimen to an elongation of 50 mm is taken as a contractive force of the specimen.

The contractibility of the gathers can be adjusted by, for example, changing at least one of the thickness, extensibility, and the number of the elastic members. The leg gathers 107 are preferably provided only in the crotch portion of the absorbent article. The term "crotch portion" as used herein denotes the portion in the middle of an absorbent article where both the lateral sides are curved inward.

The diaper 101 shown in Fig. 22 has the leg gathers and two pairs of opposing standing gathers. This configuration may be replaced with a structure having only two or more pairs of opposing standing gathers with no leg gathers. In Fig. 23, for example, two pairs of standing gathers, a first pair of standing gathers 161 and a second pair of standing gathers 162, are used. In this configuration, too, it is preferred that the contractive force of the standing gathers descend laterally from outside to inside of the diaper 101 for the same reason as mentioned.

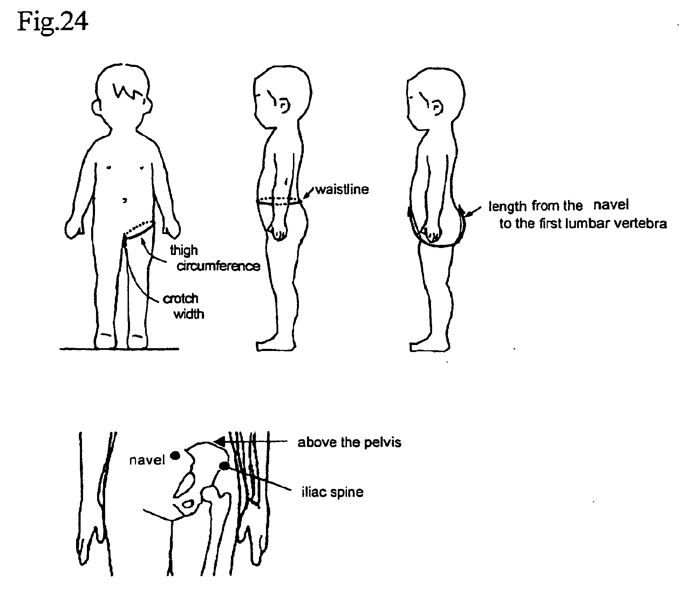

The embodiment shown in Figs 24 through 26 will now be described. As stated, the absorbent article of the present invention that uses the absorbent member having a web of continuous fibers is capable of controlling a humidity rise inside the diaper. The effect of controlling a humidity rise is more appreciable in an absorbent article that fits better. The present embodiment is pertinent to a pull-on type disposable diaper that fits snugly on the wearer's body.

In the present embodiment, the following design approach is taken to make a diaper that provides a snugger fit.

Step 1:

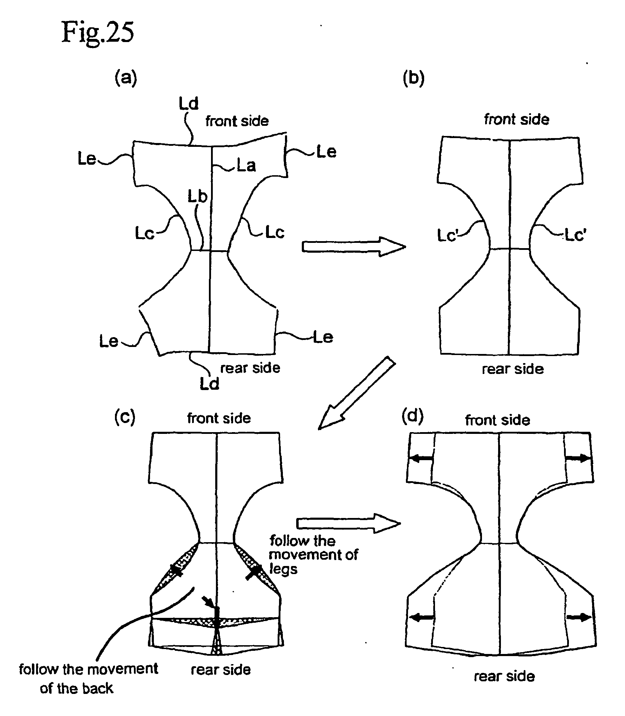

Sheeting broad enough compared with a flat out contour of an absorbent article is directly applied to the body of a wearer (e.g., an infant) to do draping. The sheeting on the body is marked with lines indicating the waist, the thigh circumference at the groin (the thigh circumference indicated in Fig. 24), and the side seam. The resulting pattern, which is taken as a base pattern for planar design, is scanned into a CAD program and then added corrections. An example of base patterns thus created by transferring the body contour is shown in Fig. 25(a). In the figure, vertical line segment La indicates the length along the body from the navel to the first lumbar vertebra of the wearer (see Fig. 24); horizontal line segment Lb that is perpendicular to the vertical line segment La indicates the position in the longitudinal direction at which the distance between the left and right thigh circumferential lines is the least (corresponding to the position in the longitudinal direction at which the crotch width of the wearer is the least) and the least width at that position; curve Lc indicates the thigh circumference at the groin; curve Ld represents the waistline passing through the navel and the first lumbar vertebra; and curve Le is the side seam line (the ridge line at the side of the wearer). In this way, a base pattern is taken from a plurality of wearers.

Step 2:

Corrections are given to each base pattern according to a given rule to correct light and left inequality. By way of example, corrections are not made to the lengths and positions of the vertical and horizontal line segments La and Lb and the shape and length of the one of the two curves Lc on the right and left of the vertical line segment La that is more reliable. The one of the two curves Lc that is more reliable is copied axisymmetrically about the vertical line segment La to give a curve Lc', which replaces the other curve Lc that is less reliable. With respect to the opposing curves Le in each of the longitudinal end portions of the base pattern, the shape and length of the longer one of the opposing curves Le are corrected to those of the shorter one. Each of the front and rear waistlines is divided into the right and left segments, which are then averaged. Fig. 25(b) represents a thus corrected base pattern.

Step 3:

The corrected base patterns are divided into groups according to age (of months), sex, etc. and averaged per group to prepare a master pattern for each group. All the available corrected base patterns may be averaged to make a sole master pattern.

Step 4:

Wearers are let move freely, and their behavior was recorded on videotape, etc. The body movements frequently shown and how the wearer's skin extends and contracts with such movements are observed. As a result of observation and analysis on the children's behavior, it has been revealed that children of age who wear a pull-on absorbent article (particularly pull-on diaper) frequently show actions such as crawling, changing from standing to sitting or vice versa, bending forward while sitting, and running about. It has also been found that the skin frequently extends and contracts in the back and the groins in crawling, in the back and the below-waist part of the stomach side in changing from standing to sitting or vice versa, in the back in forward bending while sitting, and in the groins in running about.

Step 5:

To each of the master patterns (or the single master pattern) obtained in step 3 is added correction reflecting the findings about the skin extension and contraction ascertained in step 4. The correction is made for the purpose of securing a region that can follow the skin extension and contraction. Fig. 25(c) illustrates a revised master pattern obtained by adding cross-hatched regions to the master pattern in order to afford to an absorbent article regions that can follow extension and contraction of the wearer's skin in the back and the groins. The description here does not enter into a region that can follow the skin's extension and contraction in the below-waist part of the stomach side of a wearer because the region is included in a region for imparting ease of putting on (ease of diapering) a wearer, which will be discussed in the next step.

Step 6:

To the revised master pattern is added correction for imparting ease of putting on (ease of diapering) a wearer. Fig. 25(d) shows a pattern obtained by enlarging the revised master pattern in the width direction at a prescribed ratio. The result of that correction is the final pattern furnishing the planar shape of a diaper.

Step 7:

Layout of elastic members on the final pattern is designed taking into account the skin's extension and contraction ascertained in step 4. The layout of elastic members should be designed such that regions capable of elastically extending and contracting following the skin's extension and contraction may be provided while taking leakage protection into consideration as has been in conventional diaper design. Layout design of other necessary members is then decided to complete designing a disposable absorbent article.

A pull-on disposable diaper designed based on the aforementioned design approach which satisfies one or more than one requirements (a) to (e) below provides an extremely improved fit against the wearer's body. Combinations of two or more requirements include a combination of any two of requirements (a) to (e) (hereinafter referred to as a combination (1), i.e., (a)+(b), (a)+(c), (a)+(d), (a)+(e), (b)+(c), (b)+(d), (b)+(e), (c)+(d), (c)+(e), and (d)+(e)), a combination of the combination (1) plus one of the rest (hereinafter referred to as a combination (2)), a combination of the combination (2) plus one of the rest (hereinafter referred to as a combination (3)), and a combination of the combination (3) plus the remaining one.

Requirements:

(a): An absorbent article has a waist elastic member disposed in the front and rear waist portions in the circumferential direction of the waist opening. The tensile load required to laterally stretch the waist portions to 50% of the maximum elongation is 2 to 5N.

(b): An absorbent article has a pair of elasticized regions provided outboard of the side edges of the absorbent member in the front below-waist portion. The elasticized regions are extensible in a direction 45° from the longitudinal direction of the absorbent article. The tensile load required to laterally stretch the front and rear below-waist portions to 50% of the maximum elongation is preferably 1 N or less.

(c): An absorbent article has an elasticized region between the absorbent member and the edges of the waist opening (or the longitudinal ends in the case of a flat type disposable absorbent article) in the rear portion. The edge of the waist opening in the rear portion preferably protrudes upward.

(d): An absorbent article has a waist elastic member disposed at a position that goes above the iliac spine of a wearer while worn.

(e): An absorbent article has an elasticized region near the leg opening in the rear portion. The elasticized region is extensible in the direction perpendicular to the edge of the leg opening in the rear portion. The edge of the leg opening in the rear portion preferably protrudes to the center of the leg opening.

The pull-on disposable diaper designed by the above-described design method hardly slides down and snugly fits on a wearer's body without excessively constricting.

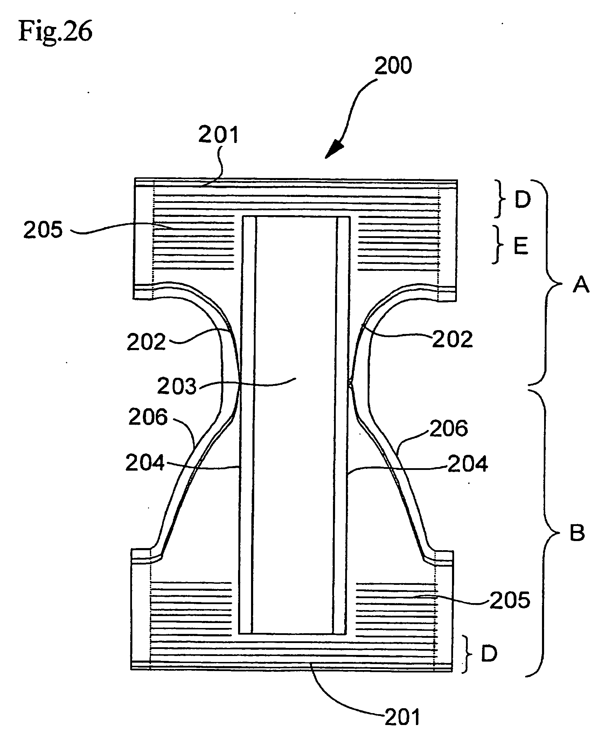

Fig. 26 depicts a pull-on disposable diaper according to the present embodiment in its flat-out, uncontracted state. The constriction by the diaper 200 illustrated in Fig. 26 is preferably such that a tensile load required to extend the front and rear waist portions D in the diaper width direction up to 50% of the maximum elongation is 2 to 5 N, more preferably 3 to 4 N. The tensile load at 50% of the maximum elongation is measured as follows.

Method of measuring tensile load in waist portions:

The front and rear waist portions as linked to each other are cut out of a diaper to make an annular specimen. The specimen is placed on a horizontal surface with the outer side of either one of the waist portions in contact with the horizontal surface. The end-to-end length of the specimen (i.e., the length between the right and left side joints) in a relaxed state is measured to give an initial length. The specimen is set between chucks of a Tensilon tensile tester (RTC-1150A, from Orientech Co., Ltd.) and extended in the direction corresponding to the diaper width direction at a rate of 300 mm/min up to its designed dimension shown in Fig. 26. The designed dimension is the same as the dimension of the flat-out specimen with any influences of the elastic members excluded. When the designed dimension of the waist portion differs between the front portion A and the rear portion B, the average of the two is used. The elongation at the designed dimension [(stretched length - initial length)/initial length x 100] is taken as the maximum elongation. The tensile load applied is read out when the specimen is extended to 50% of the maximum elongation.

Furthermore, the tensile load required to laterally stretch the front and rear below-waist portions E, E (see Fig. 26) to 50% of the maximum elongation is preferably 1 N or less. The tensile load of the below-waist portions is measured as follows. Method of measuring tensile load in below-waist portions:

The front and rear below-waist portions as linked to each other are cut out of a diaper to make an annular specimen. The specimen is placed on a horizontal surface with the outer side of either one of the below-waist portions in contact with the horizontal surface. The end-to-end length of the specimen (i.e., the length between the right and left side joints) in a relaxed state is measured to give an initial length. The specimen is set between chucks of a Tensilon tensile tester (RTC-1150A, from Orientech Co., Ltd.) and extended in the direction corresponding to the diaper width direction at a rate of 300 mm/min up to its designed dimension shown in Fig. 26. The designed dimension is the same as the dimension of the flat-out specimen with any influences of the elastic members excluded. When the designed dimension of the below-waist portion differs between the front portion A and the rear portion B, the average of the two is used. The elongation at the designed dimension [(stretched length - initial length)/initial length x 100] is taken as the maximum elongation. The tensile load applied is read out when the specimen is extended to 50% of the maximum elongation.

A pull-on disposable diaper was made as a prototype according to the present embodiment, and a pressure sensor was inserted between the below-waist portion E of the front portion A and the skin of a wearer to measure the pressing force against the skin. The pressing force was found to be 2 g/cm$^2$ or less, proving the diaper far less constrictive than a conventional pull-on diaper having a below-waist gather (the pressing force of which is 7 g/cm$^2$ or more).

When the diaper 200 shown in Fig. 26 is stretched at its waist, the ratio of the circumference of the waist opening edge in its state stretched to the limit to that in its relaxed state is preferably 1.4 to 2.2, more preferably 1.6 to 2.0. The diaper 200 has a waist elastic member 201 disposed in each of the front and rear portions A and B at a position corresponding to above the iliac spine of a wearer (i.e., a position located at a height above the iliac spine of an upstanding wearer) while the diaper is worn.

The diaper 200 has a leg elastic member 202 disposed along the periphery of each leg opening. The diaper 200 also has an elasticized region extensible in the diaper width direction provided outboard of each side edge 204 of the absorbent member 203 in the below-waist portion E. The below-waist elastic members 205 are separately provided on both sides of the absorbent member 203. The below-waist elastic members 205 on the right and left hand sides of the absorbent member 203 may be continuous to each other across the absorbent member 203.

The edge of the leg openings in the rear portion B is curved to protrude toward the center of the leg openings as shown in Fig. 26.

While the present invention has been described based on its preferred embodiments, it should be understood that the present invention is not limited thereto. By way of example, the embodiments illustrated in Figs. 20 to 26 can be applied to the conventional disposable diaper with side panels described with reference to background art. Because a disposable diaper with side panels generally easily comes into close contact with a wearer's body, a humidity rise inside the diaper tends to occur. Therefore, application of the embodiments shown in Figs. 20 to 26 to such a diaper is beneficial in controlling a humidity rise inside the diaper.

Where the embodiments of Figs. 20 to 26 are applied to a disposable diaper with side panels, the shape of the side panels is preferably designed according to the aforementioned design method. Extensible materials constructing the side panels typically include composites made of an elastomeric material and woven or nonwoven fabric, preferably nonwoven fabric. Examples of the elastomeric material include polystyrene, isoprene or polybutadiene block copolymers (e.g., styrene-ethylbutadiene-styrene and styrene-isoprene-styrene), ethylene copolymers, natural rubber, urethane, a vinyl acetate-ethylene copolymer, an ethyl acetate-ethylene copolymer, an acrylic acid-ethylene copolymer, methyl acrylate-ethylene copolymer, and an ethylene-propylene copolymers. The elastomeric material is processed into the form of a film or a fiber aggregate. The nonwoven fabric can be made of single-component fibers of thermoplastic resins, such as polyethylene, polypropylene, and polyethylene terephthalate or conjugate fibers made of two or more of these resins. Examples of the nonwoven fabrics are thermal-bonded nonwoven fabrics, spun-bonded nonwoven fabrics, spunbonded-meltblown-spunbonded nonwoven fabrics, spunbonded-meltblown-meltblown-spunbonded nonwoven fabrics, needle punched nonwoven fabrics, and hydroentangled nonwoven fabrics. Extensibility and contractibility can be imparted to the elastomeric material/nonwoven fabric composite by a method including combining an elastomer and the nonwoven fabric with no stretch and slitting the nonwoven fabric or applying a kind of breaking treatment to the nonwoven fabric or a method including combining nonwoven fabric with a tensioned elastomer. Whatever material and method are used, the extensible material is desirably breathable.

The present invention is applicable to not only disposable diapers but also other absorbent articles, such as various types of sanitary napkins and incontinence pads. Application to pull-on type absorbent articles including panty-type sanitary napkins is especially effective.

The present invention will now be illustrated in greater detail with reference to Examples, but it should be understood that the present invention is not construed as being limited thereto.


EXAMPLE 1-1


A tow of crimped, continuous cellulose acetate fibers was prepared. The individual continuous fibers had a fineness of 2.1 dtex, and the liner density of the tow was 25,000 dtex. The tow was fed under tension and opened in an air opening apparatus. The tow-opening web was passed between a roller having a large number of discs arrayed around its periphery in an axial direction at a given interval and a smooth anvil roller, between which the web was combed. The width of the web was adjusted to 100 mm. The running speed of the web was slowed down, and the web was transferred onto a vacuum conveyor. The web on the vacuum conveyor was released from the tension to develop the crimp. The continuous fibers of the web had a crimp percentage of 30%, and the number of the crimps per centimeter was 15. The fiber interstices in the web were thus broadened to help a superabsorbent polymer enter and to make the web bulkier thereby to improve polymer supporting capability. A superabsorbent polymer was then sprinkled on the opened web and embeddedly supported thereby. The weights of the web and the superabsorbent polymer were 25 g/m$^2$ and 110 g/m$^2$, respectively.

Separately, 100 parts by weight of opened fluff pulp and 100 parts by weight of a superabsorbent polymer were uniformly mixed in an air stream and deposited on a T-shaped mold to obtain a fiber accumulated member weighing 300 g/m$^2$. The vertical part of the T-shaped mold measured 100 mm by 100 mm, and the horizontal part measured 100 mm by 125 mm. The fluff pulp and superabsorbent polymer of the fiber accumulated member each weighed 150 g/m$^2$.

The web was superposed on the fiber accumulated member, and the resulting structure was wrapped in tissue paper having a grammage of 16 g/m$^2$ on which a hot melt adhesive had been applied by spraying to obtain an absorbent member having the structure illustrated in Figs. 1 and 2. The absorbent member had a weight of 477 g/m$^2$ and a thickness of 2.1 mm. The opened web had a weight of 25 g/m$^2$.

An air-through nonwoven fabric weighing 25 g/m$^2$ was used as a topsheet. The air-through nonwoven fabric was made of linear low density polyethylene sheath/polypropylene core conjugate fiber (thickness: 2.1 dtex; having been treated with a surface active agent to have liquid permeability). A composite of a porous film weighing 20 g/m$^2$ and a spun-bonded polypropylene nonwoven fabric weighing 20 g/m$^2$, bonded with 1.5 g/m$^2$ of a hot melt adhesive, was used as a backsheet. The porous film was produced by blown-film extruding a uniform mixture of 100 parts by weight of linear low density polyethylene (density: 0.925 g/cm$^2$), 150 parts by weight of calcium carbonate, and 4 parts by weight of an ester compound as a third component and longitudinally stretching the blown film to double the length. A disposable diaper was made in an otherwise conventional manner to manufacture disposable diapers. The absorbent member was disposed with the orientation direction of the web coinciding with the longitudinal direction of the diaper.

EXAMPLE 1-2

A superabsorbent polymer was embeddedly supported in a tow-opening web in the same manner as in Example 1-1, except for changing the amount of the sprinkled superabsorbent polymer to 260 g/m$^2$. Separately, opened fluff pulp was air-laid on the same T-shaped mold as used in Example 1-1 to obtain a fiber accumulated member weighing 100 g/m$^2$. The web was superposed on the fiber accumulated member, and the resulting structure was wrapped in a hydrophilized spunbonded-meltblown-spunbonded nonwoven fabric (SMS) weighing 16 g/m$^2$ to obtain an absorbent member. The resulting absorbent member had a weight of 477 g/m$^2$ and a thickness of 2.0 mm. A disposable diaper was made in otherwise the same manner as in Example 1-1.

EXAMPLE 1-3

An absorbent member and an absorbent article were made in the same manner as in Example 1-1, except for changing the adjusted width of the tow-opening web to 125 mm and the amount of the sprinkled superabsorbent polymer to 90 g/m$^2$.

EXAMPLE 1-4

An absorbent member and an absorbent article were made in the same manner as in Example 1-3, except for inverting the positions of the web having the superabsorbent polymer embeddedly supported therein and the fiber accumulated layer of the fluff pulp/superabsorbent polymer mixture.

EXAMPLE 1-5

A disposable diaper was obtained in the same manner as in Example 1-1, except that the continuous fibers constituting the web had a fineness of 6.7 dtex, a crimp percentage of 24%, and the number of crimps per cm of 10; the tow had a linear density of 17,000 dtex; and the web weighed 30 g/m$^2$

COMPARATIVE EXAMPLE 1-1

A hundred parts by weight of opened fluff pulp and 100 parts by weight of a superabsorbent polymer were uniformly mixed in an air stream and deposited to obtain a fiber accumulated member weighing 520 g/m$^2$. The amounts of the fluff pulp and the superabsorbent polymer were both 260 g/m$^2$. The fiber accumulated member was wrapped in tissue paper to obtain an absorbent member. The fiber accumulated member and the tissue paper were adhered with 5 g/m$^2$ of a hot melt adhesive applied therebetween by spraying. The absorbent member had a weight of 562 g/m$^2$ and a thickness of 4.3 mm. A disposable diaper was made in otherwise the same manner as in Example 1-1.

COMPARATIVE EXAMPLE 1-2

A fiber accumulated member weighing 300 g/m$^2$ was obtained by uniformly mixing 100 parts by weight of opened fluff pulp and 100 parts by weight of a superabsorbent polymer in an air stream in the same manner as in Comparative Example 1-1. The amounts of the fluff pulp and the superabsorbent polymer were both 150 g/m$^2$. An absorbent member was obtained in otherwise the same manner as in Comparative Example 1-1. The resulting absorbent member had a weight of 342 g/m$^2$ and a thickness of 2.6 mm. A disposable diaper was made in the same manner as in Example 1-1, except for using the resulting absorbent member.

COMPARATIVE EXAMPLE 1-3

In an attempt to make a fiber accumulated member weighing 350 g/m$^2$ in the same manner as in Comparative Example 1-1, 100 parts by weight of opened fluff pulp and 250 parts by weight of a superabsorbent polymer were uniformly mixed in an air stream. However, the fluff pulp failed to support the superabsorbent polymer, only to result in an unsatisfactory absorbent member.

Performance Evaluation:

The absorbent members obtained in Examples and Comparative Examples were measured for absorption capacity and evaluated for structural stability and flexibility in accordance with the methods below. The results obtained are shown in Table 1 below.

(1) Absorption capacity

The absorbent member was fixed to an inclined plate set at 45°. A given amount of physiological saline was poured at a given time interval at a position 200 mm below the upper end of the absorbent member. The amount of physiological saline that had been poured until it began to leak from the lower end of the absorbent member was compared. Taking the absorption capacity of Comparative Example 1-1 as 1.0, the results were expressed relatively by calculation using equation:

$$\text{Absorption capacity (relative)} = \text{absorption capacity of sample/absorption capacity of}$$
$$\text{Comparative Example 1-1}$$

(2) Structural stability

(2-1) While dry

An absorbent member measuring 100 mm by 200 mm was cut into halves along the lateral centerline to make a piece measuring 100 mm by 100 mm. The piece was shaken 20 times with an amplitude of 5 cm at a rate of one shake per second with the cut area down. The polymer particles fallen from the cut area was weighed. The polymer supporting properties of the absorbent member was rated based on the ratio of the fallen polymer to the total polymer as follows.

A: The ratio of the fallen polymer is within 10%.
B: The ratio of the fallen polymer is higher than 10% and not higher than 25%.
C: The ratio of the fallen polymer is higher than 25%.

(2-2) While wet

A 100 mm by 200 mm cut piece of an absorbent member was almost uniformly impregnated with 200 g of physiological saline and then gently lifted to see if the absorbent member suffered from destruction by visual observation.
Apart from the above evaluation, the fallen superabsorbent polymer was weighed. The weight was divided by a water retention per unit weight of the superabsorbent polymer previously determined by a centrifugal dewatering method to obtain the dry weight of the fallen superabsorbent polymer. The ratio of the fallen superabsorbent polymer was calculated therefrom relative to the total amount of the polymer having been supported. The polymer supporting properties were rated based on the calculated ratio of the superabsorbent polymer according to the criteria described below.
The amount of the superabsorbent polymer that had been supported was determined as follows. An absorbent member to be analyzed, the weight of which has been measured, is immersed in an aqueous ascorbic acid solution and exposed to sunlight for a sufficient period of time to dissolve the superabsorbent polymer completely, followed by washing with water. The decomposition of the superabsorbent polymer and washing of the absorbent member with water are repeated until the superabsorbent polymer is completely dissolved. The absorbent member is then dried and weighed. The weight of the dried absorbent member is subtracted from the weight of the absorbent member before the superabsorbent polymer decomposition to give the amount of the superabsorbent polymer that has been present in the absorbent polymer.

A: The ratio of the fallen polymer is 10% or lower. No structural destruction of the absorbent member is observed.
B: The ratio of the fallen polymer is higher than 10% and not higher than 25%. No structural destruction of the absorbent member is observed.
C: The ratio of the fallen polymer is higher than 25%, or structural destruction of the absorbent member is observed.

Flexibility

Flexibility of an absorbent member was evaluated by the use of a handle-o-meter. The smaller the "handle" value as measured with a handle-o-meter, the easier to put on and the snugger the fit. Measurement with a handle-o-meter is carried out as follows in accordance with JIS L1096 (stiffness and softness measuring method). A specimen measuring 50 mm wide and 150 mm long is placed on the platform having a 60 mm wide slot with the length perpendicular to the slot. The force required for a 2 mm thick penetrator blade to force the center of the specimen into the slot is read. In the present invention, a handle-o-meter HOM-3 available from Daiei Kagaku Seiki Co., Ltd. was used. The measurement was made on three points to obtain an average. The flexibility was rated as follows.

A: The "handle" is 2N or less.
B: The "handle" is more than 2N and not more than 4N.
C: The "handle" is more than 4N.

TABLE 1

| | | Example | | | | | Comparative Example | | |
|---|---|---|---|---|---|---|---|---|---|
| | | 1-1 | 1-2 | 1-3 | 1-4 | 1-5 | 1-1 | 1-2 | 1-3 |
| Upper Layer | Fineness of Single Continuous Fiber | 2.1 | 2.1 | 2.1 | 2.1 | 6.7 | - | - | 0 |
| | Weight of Continuous Fibers (g/m$^2$) | 25 | 25 | 25 | - | 30 | - | - | - |
| | Width of Tow-opening Web of Continuous Fibers (mm) | 100 | 100 | 125 | - | 100 | - | - | - |
| | Weight of Pulp (g/m$^2$) | 0 | 0 | 0 | 150 | 0 | - | - | - |
| | Weight of Superabsorbent Polymer (g/m$^2$) | 110 | 260 | 90 | 150 | 110 | - | - | - |
| Lower Layer | Weight of Pulp (g/m$^2$) | 150 | 100 | - | - | 150 | 260 | 150 | 150 |
| | Fineness of Single Continuous Fiber | 2.1 | 2.1 | 2.1 | 2.1 | 6.7 | - | - | - |
| | Weight of Continuous Fibers (g/m$^2$) | - | - | 25 | 25 | - | - | - | - |
| | With of Tow-opening Web of Continuous Fibers (mm) | - | - | 125 | 125 | - | - | - | - |
| | Weight of Superabsorbent Polymer (g/m$^2$) | 150 | 0 | 90 | 90 | 150 | 260 | 150 | 260 |

(continued)

| | | | Example | | | | | Comparative Example | | |
|---|---|---|---|---|---|---|---|---|---|---|
| | | | 1-1 | 1-2 | 1-3 | 1-4 | 1-5 | 1-1 | 1-2 | 1-3 |
| Absorbent Member | Thickness (mm) | | 2.1 | 2.0 | 1.9 | 2.1 | 1.8 | 4.3 | 2.7 | - |
| | Absorption Capacity | | 1.0 | 1.0 | 1.0 | 1.0 | 1.0 | 1.0 | 0.6 | - |
| | Structural Stability | Dry | A | A | A | A | A | A | A | C |
| | | Wet | A | A | A | A | A | A | A | - |
| | Flexibility | | A | A | A | A | A | C | A | - |
| Evaluation in Compara. Example 1-3 was partial because of the failure to obtain a satisfactory absorbent member. | | | | | | | | | | | |

As is apparent from the results in Table 1, the absorbent members of Examples prove structurally stable although they have a smaller fibrous material content than those of Comparative Examples. They also prove highly absorptive while they are thin, light weight, and flexible.

EXAMPLE 2-1

A tow of crimped, continuous cellulose acetate fibers was prepared. The continuous fibers had a fineness of 2.1 dtex. The linear density of the tow was 25,000 dtex. The tow was fed under tension and opened in an air opening apparatus. The tow-opening web was passed between a roller having a large number of discs arrayed around its periphery in an axial direction at a given interval and a smooth anvil roller, between which the web was combed. The width of the web was adjusted to 100 mm. The running speed of the web was slowed down, and the web was transferred onto a vacuum conveyor. The web on the vacuum conveyor was released from the tension to develop the crimp. By these operations, there were formed higher fiber content regions and lower fiber content regions in the web of continuous fibers. The operations also resulted in adequate interfiber distances and thickness of the web for supporting a superabsorbent polymer. The continuous fibers of the web had a crimp percentage of 30%, and the number of the crimps per cm was 15. The average weight per unit area of the web was 26 g/m$^2$. A superabsorbent polymer was then sprinkled on the web to be embeddedly supported. The weight of the superabsorbent polymer supported was 260 g/m$^2$. The resulting web was wrapped in tissue paper having a grammage of 16 g/m$^2$, on which 5 g/m$^2$ of a hot melt adhesive had been sprayed, to prepare an absorbent member.
A disposable diaper was made in a usual manner using the same topsheet and backsheet as in Example 1-1. The absorbent member was disposed with the orientation direction of the web coinciding with the longitudinal direction of the diaper.

EXAMPLE 2-2

A tow-opening web having higher and lower fiber content regions and having superabsorbent polymer particles embeddedly supported therein was prepared in the same manner as in Example 2-1, except for changing the amount of the superabsorbent polymer to be sprinkled to 110 g/m$^2$. A fluff pulp/superabsorbent polymer fiber accumulated member having a weight of 300 g/m$^2$ was superposed on the web. The fiber accumulated member was obtained by uniformly mixing 100 parts by weight of opened fluff pulp and 100 parts by weight of a superabsorbent polymer in an air stream. The weights of the fluff pulp and the superabsorbent polymer in the fiber accumulated member were both 150 g/m$^2$. The resulting structure was wrapped in a hydrophilized spunbonded-meltblown-spunbonded nonwoven fabric (SMS) weighing 16 g/m$^2$ to obtain an absorbent member. A disposable diaper was made in otherwise the same manner as in Example 2-1.

EXAMPLE 2-3

An absorbent structure was prepared in the same manner as in Example 2-1, except that the continuous fibers had a fineness of 6.7 dtex, the tow had a linear density of 17,000 dtex, the continuous fibers had a crimp percentage of 24% and the number of crimps of 10 per centimeter, and the web had an average weight of 30 g/m$^2$. A disposable diaper was made in otherwise the same manner as in Example 2-2.

COMPARATIVE EXAMPLE 2-1

A hundred parts by weight of opened fluff pulp and 100 parts by weight of a superabsorbent polymer were uniformly mixed in an air stream to obtain a fiber accumulated member weighing 520 $g/m^2$. The weights of the fluff pulp and the superabsorbent polymer were both 260 $g/m^2$. The fiber accumulated member was wrapped in tissue paper having a grammage of 16 $g/m^2$ to obtain an absorbent member. The fiber accumulated member and the tissue paper were adhered with 5 $g/m^2$ of a hot melt adhesive sprayed therebetween. A disposable diaper was made in otherwise the same manner as in Example 2-1.

COMPARATIVE EXAMPLE 2-2

An air-through nonwoven fabric weighing 30 $g/m^2$ (made of linear low density polyethylene sheath/polypropylene core conjugate fiber having a thickness of 5.6 dtex, having been hydrophilized with a surface active agent) was superposed on the skin facing side of the fluff pulp/superabsorbent polymer fiber accumulated member prepared in Comparative Example 2-1. A disposable diaper was made in otherwise the same manner as in Example 2-1.

Evaluation of Performance:

The absorbent members of the diapers obtained in Examples and Comparative Examples were evaluated for absorption capacity, structural stability, and flexibility in accordance with the same methods as followed in Example 1-1. Additionally, the disposable diapers were evaluated for dry feeling after liquid absorption. The results obtained are shown in Table 2 below. The absorption capacity was expressed relatively taking the result of Comparative Example 2-1 as 1.0.

Dry feeling:

Three 40 g portions of colored physiological saline (colored with FD & C Red No. 1 dissolved at a 0.05% concentration) were poured on the diaper placed horizontally at a 5 minute interval. Ten mothers were asked to observe the surface condition of the diaper after absorption and speak of their visual impression. They were asked to judge the visual sensation of dryness using Example 2-1 as a benchmark. The amount of residual liquid in the topsheet was then measured as follows. The topsheet was stripped off the diaper that had been judged for dryness by the mothers and weighed (W1). The liquid remaining in the topsheet was completely wiped off with facial tissue, and the thus dried topsheet was weighed again (W2). The amount of the residual liquid was calculated by equation:

$$\text{Amount of residual liquid (g)} = W1 - W2$$

The dry feeling of the diaper was rated from the mothers' visual impression and the amount of residual liquid according to the following criteria.

A: More than 5 mothers answer that the diaper gives a visual sensation of dryness, and the amount of residual liquid is 0.3 g or less.
B: Less than 6 mothers answer that the diaper gives a visual sensation of dryness, or the amount of residual liquid is more than 0.3 g.
C: More than 4 mothers answer that the diaper visually lacks a dry feeling, and the amount of residual liquid is more than 0.3 g.

TABLE 2

| | | Example | | | Comparative Example | |
|---|---|---|---|---|---|---|
| | | 2-1 | 2-2 | 2-3 | 2-1 | 2-2 |
| Web | Fineness of Single Continuous Fiber | 2.1 | 2.1 | 6.7 | - | - |
| | Weight of Continuous Fibers ($g/m^2$) | 26 | 26 | 30 | - | - |
| | Weight of Super-absorbent Polymer ($g/m^2$) | 260 | 110 | 110 | - | - |

(continued)

| | | Example | | | Comparative Example | |
|---|---|---|---|---|---|---|
| | | 2-1 | 2-2 | 2-3 | 2-1 | 2-2 |
| Fiber Accumulated Member | Weight of Pulp (g/m$^2$) | - | 150 | 150 | 260 | 260 |
| | Weight of 2 Super-absorbent Polymer (g/m$^2$) | - | 150 | 150 | 260 | 260 |
| Absorbent Member | Nonwoven Fabric | no | no | no | no | yes |
| | Thickness (mm) | 2.0 | 2.3 | 1.9 | 4.3 | 4.7 |
| | Absorption Capacity | 1.0 | 1.0 | 1.0 | 1.0 | 1.0 |
| | Structural Stability — Dry | A | A | A | A | A |
| | Structural Stability — Wet | A | A | A | A | A |
| | Flexibility | A | A | A | B | B |
| | Dry Feeling | A | A | A | B | A |

As is apparent from the results shown in Table 2, each of the absorbent members of Examples is, while thinner than those of Comparative Examples, equal in absorption capacity and structural stability to those of Comparative Examples. It is also seen that the absorbent members of Examples are more flexible and give a user a higher dry feeling than those of Comparative Examples.

EXAMPLE 3-1

A tow of crimped, continuous cellulose acetate fibers was prepared. The continuous fibers had a fineness of 2.1 dtex. The linear density of the tow was 25,000 dtex. The tow was fed under tension and opened in an air opening apparatus. The tow-opening web was passed between a roller having a large number of discs arrayed around its periphery in an axial direction at a given interval and a smooth anvil roller, between which the web was combed. The width of the web was then adjusted to 100 mm. The running speed of the web was slowed down, and the web was transferred onto a vacuum conveyor. The web on the vacuum conveyor was released from the tension to develop the crimp. The crimped continuous fibers in the resulting web had a crimp percentage of 30%, and the number of the crimps per centimeter was 15. The interfiber spaces in the web were thus broadened to help a superabsorbent polymer enter and to make the web bulkier thereby to improve polymer supporting capability. A superabsorbent polymer was then sprinkled on the tow-opening web in an amount of 130 g/m$^2$ to give a first web p1 having the polymer particles embeddedly supported therein. The above-described operations were conducted once more to make a second web p2 having the superabsorbent polymer particles embeddedly supported therein. The first web p1 and the second web p2 were superposed on each other with a fluff pulp layer weighing 50 g/m$^2$ interposed therebetween.

A fluff pulp layer weighing 100 g/m$^2$ was superposed on the second web p2. The resulting absorbent structure was wrapped in tissue paper having a grammage of 16 g/m$^2$, on which 5 g/m$^2$ of a hot melt adhesive had been sprayed, to prepare an absorbent member. The absorbent member was configured to be used with its outer fluff pulp layer (100 g/m$^2$) facing a garment. The absorbent member had a weight of 502 g/m$^2$ and a thickness of 2.2 mm. The tow-opening webs each weighed 13 g/m$^2$.

A disposable diaper having the structure shown in Figs. 20 and 21 was made in a usual manner, except for using the same topsheet and backsheet as used in Example 1-1. The topsheet was used after perforating with 5 mm diameter metal pins. The absorbent member was disposed with the orientation direction of the webs coinciding with the longitudinal direction of the diaper.

EXAMPLE 3-2

A web p1 having superabsorbent polymer particles embeddedly supported therein was prepared in the same manner as in Example 3-1, except for changing the amount of the superabsorbent polymer to be sprinkled to 110 g/m$^2$. A mixed structure m weighing 300 g/m$^2$ was prepared by uniformly mixing 100 parts by weight of opened fluff pulp and 100 parts by weight of a superabsorbent polymer in an air stream. The weights of the fluff pulp and the superabsorbent polymer in the mixed structure were both 150 g/m$^2$. The mixed structure m was superposed on the web p1, and the laminate was wrapped in a hydrophilized spunbonded-meltblown-spunbonded nonwoven fabric (SMS) weighing 16 g per square meter to make an absorbent member having a weight of 482 g/m$^2$ and a thickness of 2 mm. A disposable diaper was obtained in otherwise the same manner as in Example 3-1.

EXAMPLE 3-3

An absorbent member was obtained in the same manner as in Example 3-2, except that the laminate of the web p1 and the mixed structure m was perforated with 5 mm diameter metal pins. Perforation was effected in an area from 100 mm to 200 mm below the longitudinal end of the front portion of the absorbent member over a width of 50 mm. The metal pins were arranged in a staggered pattern. The distance between the tips of metal pins adjacent in the width direction and the distance between the tips of metal pins adjacent in the longitudinal direction were both 1 cm. A disposable diaper was obtained using the resulting absorbent member in the same manner as in Example 3-2.

COMPARATIVE EXAMPLE 3-1

The same as Comparative Example 2-1.

COMPARATIVE EXAMPLE 3-2

The same as Comparative Example 2-2.

COMPARATIVE EXAMPLE 3-3

A mixed structure weighing 400 g/m$^2$ was prepared by uniformly mixing 100 part by weight of opened fluff pulp and 100 parts by weight of a superabsorbent polymer in an air stream in the same manner as in Comparative Example 3-1. The weights of the fluff pulp and the superabsorbent polymer in the mixed structure were both 200 g/m$^2$. An absorbent member was made in otherwise the same manner as in Comparative Example 3-1. The absorbent member had a weight of 442 g/m$^2$ and a thickness of 3.0 mm. A disposable diaper was made in the same manner as in Comparative Example 3-1, except for using the resulting absorbent member.

COMPARATIVE EXAMPLE 3-4

A mixed structure weighing 200 g/m$^2$ was prepared by uniformly mixing 100 part by weight of opened fluff pulp and 100 parts by weight of a superabsorbent polymer in an air stream in the same manner as in Comparative Example 3-1. The weights of the fluff pulp and the superabsorbent polymer in the mixed structure were both 100 g/m$^2$. An absorbent member was made in otherwise the same manner as in Comparative Example 3-1. The absorbent member had a weight of 242 g/m$^2$ and a thickness of 1.7 mm. A disposable diaper was made in the same manner as in Comparative Example 3-1, except for using the resulting absorbent member.

Evaluation of performance:

The absorbent members of the diapers obtained in Examples and Comparative Examples were evaluated for absorption capacity, structural stability, and flexibility in accordance with the same methods as adopted in Example 1-1. In addition, air permeation resistance of the absorbent members and fit of the disposable diapers were measured or evaluated in accordance with the methods below. The results obtained are shown in Table 3 below. The absorption capacity was expressed relatively taking the result of Comparative Example 3-1 as 1.0.

(1) Air permeation resistance

An air permeation resistance of an absorbent member measuring 100 mm by 100 mm was measured with an air permeability tester KES-F8 (trade name) from Kato Tech Co., Ltd. KES-F8 sends air at a constant flow rate (4 cc/cm$^2$/sec) to a sample to measure the pressure loss, from which an air permeation resistance is calculated. The measurement was taken on five points to obtain an average.

(2) Fit

The disposable diapers prepared were worn by five babies of 7 to 14 months of age who usually wore large size pull-on diapers. The baby's mothers were asked to speak of their impression about the fit.

A: A snug fit is provided with no bagginess around the crotch.
B: A slightly snug fit is provided.
C: A snug fit is not provided. Bagginess around the crotch is felt.

TABLE 3

| | | Example | | | Comparative Example | | | |
|---|---|---|---|---|---|---|---|---|
| | | 3-1 | 3-2 | 3-3 | 3-1 | 3-2 | 3-3 | 3-4 |
| Absorbent Member | Material | continuous fiber web, pulp | continuous fiber web, pulp/ polymer mixture | continuous fiber web, pulp/ polymer mixture | pulp/polymer mixture | pulp/polymer mixture | pulp/polymer mixture | pulp/polymer mixture |
| | Wrapping Sheet | tissue paper | hydrophilized SMS | hydrophilized SMS | tissue paper | tissue paper | tissue paper | tissue paper |
| | Thickness (mm) | 2.2 | 2.0 | 2.0 | 4.3 | 2.6 | 3 | 1.7 |
| | Perforation | no | no | yes | no | no | no | no |
| | Weight of Fibers (g/m⁻) | 176 | 163 | 163 | 260 | 150 | 200 | 100 |
| | Weight of Polymer (g/m²) | 260 | 260 | 260 | 260 | 150 | 200 | 100 |
| Absorption Capacity | | 1.0 | 1.0 | 1.0 | 1.0 | 0.6 | 0.8 | 0.3 |
| Air Permeation Resistance (kPa/m·s)*2 | | 0.22 | 0.25 | 0.08 or less (less than the detection limit) | 0.53 | 0.57 | 0.60 | 0.61 |
| Flexibility | | A | A | A | C | B | C | A |
| Structural Stability | Dry | A | A | A | A | A | A | A |
| | Wet | A | A | A | A | C | A | C |
| Fit | | A | A | A | B | A | B | A |

*1: Relative value with Comparative Example 3-1 as the standard
*2: Value per 100 g superabsorbent polymer per m².

EP 1 862 156 B1

As is apparent from the results in Table 3, the absorbent members made in Examples have sufficient absorption capacity and low air permeation resistance. It is also seen that they are flexible and that they are structurally stable whether dry or wet. The diapers having the absorbent members of Examples prove to provide a good fit.

Industrial Applicability:

The present invention provides an absorbent member, of an absorbent article, with a reduced thickness and a reduced weight per unit area while securing the same level of absorption capacity as with conventional absorbent members. The absorbent member of the invention is resistant to structural destruction by active movement of a wearer. When, in particular, the absorbent member contains a superabsorbent polymer, fall-off of the polymer hardly occurs.

The present invention provides an absorbent member with improved flexibility and sufficient breathability without sacrificing absorption performance. Therefore, the absorbent article according to the present invention provides an improved fit on the body of a wearer and suppresses a rise in internal humidity while worn.

Where the continuous fiber web making up the absorbent member has higher and lower fiber content regions alternating in parallel, the absorption performance of the absorbent member can efficiently be brought out so that the entire area of the absorbent member can be made full use of to absorb body fluids. Because of the alternate higher and lower fiber content regions, the absorbent member takes on a striped appearance, which gives a user a visual sensation of dryness. Furthermore, the higher fiber content regions produce a cushioning effect, provide an improve fit, and eliminate discomfort during use, thereby endowing the absorbent article with a good-quality feel to the touch.

**Claims**

1. An absorbent article comprising an absorbent member having an absorbent core covered with a sheet of a fibrous material, the absorbent core comprising a web of hydrophilic, crimped continuous fibers and a superabsorbent polymer embeddedly supported in the web,
   the continuous fibers being oriented in one direction,
   the web has higher fiber content regions and lower fiber content regions all extending in the orientation direction of the continuous fibers, the higher and lower fiber content regions alternating in the direction perpendicular to the orientation direction of the continuous fibers.

2. The absorbent article according to claim 1, wherein the continuous fibers are oriented in the planar direction of the absorbent member.

3. The absorbent article according to claim 1, wherein the absorbent core further comprises a fiber accumulated layer or a nonwoven fabric superposed on the upper or lower side of the web, the fiber accumulated layer and the nonwoven fabric being made of natural pulp and/or synthetic fibers and containing or not containing a superabsorbent polymer.

4. The absorbent article according to claim 1, which further comprises a fiber accumulated layer or a nonwoven fabric superposed on the upper or lower side of the absorbent member, the fiber accumulated layer and the nonwoven fabric being made of natural pulp and/or synthetic fibers, containing or not containing a superabsorbent polymer, and being covered with a sheet of a fibrous material.

5. The absorbent article according to claim 1, which further comprises a fiber accumulated layer or a nonwoven fabric superposed on the upper or lower side of the absorbent member, the fiber accumulated layer and the nonwoven fabric being made of natural pulp and/or synthetic fibers and containing or not containing a superabsorbent polymer, and the absorbent member and the fiber accumulated layer or nonwoven fabric being covered as a unit with a sheet of a fibrous material.

6. The absorbent article according to claim 1, wherein the absorbent core comprises a fiber accumulated layer or a nonwoven fabric covered with the web, the fiber accumulated layer and the nonwoven fabric being made of natural pulp and/or synthetic fibers and containing or not containing a superabsorbent polymer.

7. The absorbent article according to claim 1, wherein the absorbent member has an air permeation resistance of 0.4 kPa·s/m or less per 100 g of the superabsorbent polymer per square meter, as measured according to the method described herein.

**Patentansprüche**

1. Saugfähiger Artikel mit einem saugfähigen Element, das einen saugfähigen Kern aufweist, der mit einer Bahn eines Fasermaterials bedeckt ist, wobei der saugfähige Kern ein Gewebe aus hydrophilen, gekräuselten durchgehenden Fasern und einem Superabsorber-Polymer aufweist, das im Gewebe eingebettet gehalten wird, wobei die durchgehenden Fasern in eine Richtung orientiert sind, das Gewebe Bereiche mit einem höheren Fasergehalt und Bereiche mit einem niedrigeren Fasergehalt aufweist, die sich alle in die Orientierungsrichtung der durchgehenden Fasern erstrecken, wobei sich die Bereiche mit höheren und niedrigeren Fasergehalt in die Richtung senkrecht zur Orientierungsrichtung der durchgehenden Fasern abwechseln.

2. Saugfähiger Artikel nach Anspruch 1, wobei die durchgehenden Fasern in die ebene Richtung des saugfähigen Elements orientiert sind.

3. Saugfähiger Artikel nach Anspruch 1, wobei der saugfähige Kern ferner eine Faserzusammenballungsschicht oder einen Vliesstoff aufweist, der über der Ober- oder Unterseite des Gewebes liegt, wobei die Faserzusammenballungsschicht und der Vliesstoff aus Naturzellstoff und/oder Kunstfasern bestehen und ein Superabsorber-Polymer enthalten oder kein Superabsorber-Polymer enthalten.

4. Saugfähiger Artikel nach Anspruch 1, der ferner eine Faserzusammenballungsschicht oder einen Vliesstoff aufweist, der über der Ober- oder Unterseite des saugfähigen Elements liegt, wobei die Faserzusammenballungsschicht und der Vliesstoff aus Naturzellstoff und/oder Kunstfasern bestehen, ein Superabsorber-Polymer enthalten oder kein Superabsorber-Polymer enthalten und mit einer Bahn eines Fasermaterials bedeckt sind.

5. Saugfähiger Artikel nach Anspruch 1, der ferner eine Faserzusammenballungsschicht oder einen Vliesstoff aufweist, der über der Ober- oder Unterseite des saugfähigen Elements liegt, wobei die Faserzusammenballungsschicht und der Vliesstoff aus Naturzellstoff und/oder Kunstfasern bestehen und ein Superabsorber-Polymer enthalten oder kein Superabsorber-Polymer enthalten und das saugfähige Element und die Faserzusammenballungsschicht oder Vliesstoff als Einheit mit einer Bahn eines Fasermaterials bedeckt sind.

6. Saugfähiger Artikel nach Anspruch 1, wobei der saugfähige Kern eine Faserzusammenballungsschicht oder einen Vliesstoff aufweist, der mit dem Gewebe bedeckt ist, die Faserzusammenballungsschicht und der Vliesstoff aus Naturzellstoff und/oder Kunstfasern bestehen und ein Superabsorber-Polymer enthalten oder kein Superabsorber-Polymer enthalten.

7. Saugfähiger Artikel nach Anspruch 1, wobei das saugfähige Element einen gemäß des hierin beschriebenen Verfahrens gemessenen Luftdurchgangwiderstand von 0,4 kPa·s/m oder weniger pro 100 g des Superabsorber-Polymers pro Quadratmeter aufweist.

**Revendications**

1. Article absorbant comprenant un élément absorbant avec un noyau absorbant recouvert d'une feuille de matériau fibreux, ledit noyau absorbant comportant une nappe de fibres longues hydrophiles crêpées et un polymère ultra-absorbant maintenu en étant enrobé dans la nappe, les fibres longues étant orientées dans une seule direction, la nappe présentant des zones à forte teneur en fibres et des zones à faible teneur en fibres s'étendant toutes dans la direction d'orientation des fibres longues, les zones à forte teneur en fibres et à faible teneur en fibres étant alternées dans la direction perpendiculaire à la direction d'orientation des fibres longues.

2. Article absorbant selon la revendication 1, où les fibres longues sont orientées dans la direction du plan de l'élément absorbant.

3. Article absorbant selon la revendication 1, où le noyau absorbant comporte en outre une couche de fibres emmêlées ou un tissu non tissé superposés à la face supérieure ou inférieure de la nappe, la couche de fibres emmêlées et le tissu non tissé étant en pâte à papier naturelle et/ou en fibres synthétiques, et contenant ou ne contenant pas un polymère ultra-absorbant.

**4.** Article absorbant selon la revendication 1, comprenant en outre une couche de fibres emmêlées ou un tissu non tissé superposés à la face supérieure ou inférieure de l'élément absorbant, la couche de fibres emmêlées et le tissu non tissé étant en pâte à papier naturelle et/ou en fibres synthétiques, contenant ou ne contenant pas un polymère ultra-absorbant, et étant recouverts d'une feuille de matériau fibreux.

**5.** Article absorbant selon la revendication 1, comprenant en outre comprises une couche de fibres emmêlées ou un tissu non tissé superposés à la face supérieure ou inférieure de l'élément absorbant, la couche de fibres emmêlées et le tissu non tissé étant en pâte à papier naturelle et/ou en fibres synthétiques et contenant ou ne contenant pas un polymère ultra-absorbant, et l'élément absorbant et la couche de fibres emmêlées ou le tissu non tissé étant recouverts en tant qu'unité d'une feuille de matériau fibreux.

**6.** Article absorbant selon la revendication 1, où le noyau absorbant comporte une couche de fibres emmêlées ou un tissu non tissé recouvert de la nappe, la couche de fibres emmêlées et le tissu non tissé étant en pâte à papier naturelle et/ou en fibres synthétiques et contenant ou ne contenant pas un polymère ultra-absorbant.

**7.** Article absorbant selon la revendication 1, où l'élément absorbant présente une résistance à la pénétration d'air mesurée conformément au procédé décrit inférieure ou égale à 0,4 kPa·s/m pour 100 g de polymère ultra-absorbant par mètre carré.

Fig.1

Fig.2

front side

rear side

crotch portion

Fig.3

(a)

(b)

Fig.4

Fig.5

Fig.6

Fig.7

Fig.8

Fig.9

(a)

1d

2

6

3

(b)

1e

2

6

3

(c)

1f

6a

3

2

6b

# Fig.10

### (a)

### (b)

### (c)

Fig.11

Fig.12

Fig.13

(a)

4d  4d

4c

(b)

4d  4d

4c

4e  4e

(c)

4d  4d

4f  4f

4c

(d)

4d  4d

4g

(e)

4c

4d

4d

Fig.14

(a)

(b)

Fig.15

(a)

(b)

(c)

Fig.16

Fig.17

(a)

(b)

(c)

Fig.18

Fig.19

Fig.20

Fig.21

Fig.22

Fig.23

EP 1 862 156 B1

# Fig.24

waistline

thigh
circumference

crotch
width

length from the navel
to the first lumbar vertebra

navel ● above the pelvis

iliac spine

51

# Fig.25

(a)

Ld
front side
Le
La
Lb
Lc
Lc
Le
Le
rear side
Ld

(b)

front side
Lc'
Lc'
rear side

(c)

front side
follow the
movement of
legs
follow the movement
of the back
rear side

(d)

front side
rear side

## Fig.26

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 57160457 A
- WO 200134082 A
- JP 2001276125 A
- EP 1417946 A1
- EP 0320989 A2
- JP 7024003 A
- JP 2003235889 A
- US 5865822 A